(19) Europäisches Patentamt
European Patent Office
Office européen des brevets

(11) EP 1 239 039 A1

(12) EUROPEAN PATENT APPLICATION
published in accordance with Art. 158(3) EPC

(43) Date of publication:
11.09.2002 Bulletin 2002/37

(21) Application number: 00981749.5

(22) Date of filing: 15.12.2000

(51) Int Cl.[7]: C12N 15/16, C07K 14/59, C12P 21/02, C12Q 1/68, C12N 1/15, C12N 1/19, C12N 1/21, C12N 5/00, A61K 45/00, A61P 9/12, A61P 9/04, A61P 37/04, G01N 33/15, G01N 33/50, G01N 33/53

(86) International application number:
PCT/JP00/08896

(87) International publication number:
WO 01/044475 (21.06.2001 Gazette 2001/25)

(84) Designated Contracting States:
AT BE CH CY DE DK ES FI FR GB GR IE IT LI LU MC NL PT SE TR
Designated Extension States:
AL LT LV MK RO SI

(30) Priority: 17.12.1999 JP 35870799
18.02.2000 JP 2000046825

(71) Applicant: Takeda Chemical Industries, Ltd.
Osaka-shi, Osaka 541-0045 (JP)

(72) Inventors:
- HINUMA, Shuji
Tsukuba-shi, Ibaraki 305-0821 (JP)
- FUKUSUMI, Shoji
Tsukuba-shi, Ibaraki 305-0044 (JP)
- FUJII, Ryo
Tsukuba-shi, Ibaraki 305-0821 (JP)
- HOSOYA, Masaki
Tsuchiura-shi, Ibaraki 300-0007 (JP)

(74) Representative: Lewin, John Harvey
Takeda Patent Office,
11-12 Charles II Street
London SW1Y 4QU (GB)

# (54) NOVEL POLYPEPTIDE AND DNA THEREOF

(57) The present invention provides novel polypeptides having physiological activity related to anterior pituitary hormones (such as LH, FSH, and TSH), DNAs coding for such polypeptides, methods for screening compounds or their salts which promote or inhibit the activity of the polypeptides of the present invention, and the like.

The polypeptides of the present invention have physiological activity related to anterior pituitary hormones (such as LH, FSH, and TSH), and can thus be used as a drug for the treatment of hypertension, autoimmune diseases, cardiac failure, and the like. The polypeptides of the present invention are also useful as a reagent for screening compounds or their salts which promote or inhibit the activity of the polypeptides of the present invention; and said compounds obtained by such screening are promising as a drug for the prevention and treatment of hypertension, autoimmune diseases, cardiac failure, and the like. Antibodies against the polypeptides of the present invention are also capable of specifically recognizing the polypeptides of the present invention, and can thus be used for quantification and the like of the polypeptides of the present invention in samples.

EP 1 239 039 A1

## Description

### TECHNICAL FIELD

The present invention relates to a novel pituitary polypeptide, a partial peptide thereof, a DNA coding therefor, or the like, and more particularly relates to an entirely novel polypeptide characterized by having activity in itself or as a result of the subunit structure formation.

### BACKGROUND ART

The pituitary gland, which is an endocrine organ located immediately below the brain, secretes a variety of physiologically active substances and hormones, and plays a central role in the endocrine system. Structurally, the pituitary gland is divided into the anterior, intermediate, and posterior lobes, each of which secretes specific hormones to regulate the functions of organisms, such as metabolism, growth, reproduction, homeostatic maintenance, neurological activity, and selfdefence. The secretion of such hormones is regulated by diverse factors such as hormones released by the hypothalamus. Hormones secreted by the pituitary gland bind to specific receptors on the cell membranes of various tissues in order to transmit signal to cells. The structure of most of these hormones has thus far been determined upon their isolation from tissue extracts or the like on the basis of their physiological activity. More recently, receptors are now being used to isolate a variety of physiologically active substances and hormones from tissue extracts or the like.
Meanwhile, rapid progress in recent genome and cDNA sequence analysis has made an enormous wealth of DNA information available. It is assumed that such DNA contains sequences coding for physiologically active substances which have thus far remained unknown. However, searches for unknown polypeptides having physiological activity such as hormones on the basis of genomic DNA or expressed sequence tags (EST) often result in the discovery of DNA sequences which are resembling those of known physiologically active substances in completely unrelated non-translated regions and protein genes. Furthermore, because of the potential presence of pseudogenes, it is difficult to ascertain which among them are actually physiologically active polypeptides.
Anterior pituitary hormones known at present include prolactin (PRL), growth hormone (GH), adrenocorticotrophic hormone (ACTH), thyroid stimulating hormone (TSH), follicle-stimulating hormone (FSH), and luteinizing hormone (LH). TSH, FSH, and LH in particular among these are glycoprotein hormones having alpha and beta subunit structures. Although these hormones share the same alpha chain, there are considerable structural differences in the beta chain, which determines the specific biological activity of these hormones. The expression of activity normally requires a subunit structure consisting of both an alpha and beta chain. The alpha chain has five disulfide bonds and two sugar-binding sites, while the beta chain has six disulfide bonds and one sugar-binding site. Sugar chains are also believed to play a major role in the expression of activity (A.J. Lapthorn et al., *Nature* **369**:455-461 (1994)).
There are a number of reports on the physiological activity of these anterior pituitary hormones. FSH and LH are factors essential to gonadal growth and differentiation in mammals. FSH promotes spermatogenesis in the testes and the development of ovarian follicles. LH promotes the testicular androgen secretion and ovarian ovulation. TSH is a hormone essential for the regulation of the thyroid function.
As noted above, the extremely important physiological activity of the pituitary glycoprotein hormones has been amply reported. However, no pituitary glycoprotein hormones other than TSH, FSH, and LH are known in mammals.
There is thus a need to find unknown pituitary polypeptides and the genes coding for them, in order to develop drugs for the prevention and treatment of diseases using such peptides per se, or agonists or antagonists of the peptides.

### DISCLOSURE OF THE INVENTION

As a result of extensive research to resolve the aforementioned issues, the inventors successfully cloned a cDNA having a novel base sequence by preparing primers for RT-PCR using human pituitary poly(A) $^+$RNA as template. The inventors thus discovered that the polypeptide encoded by the resulting cDNA was a novel, useful pituitary glycoprotein hormone, and perfected the present invention as a result of further extensive research based on these findings.
That is, the present invention relates to:

(1) a polypeptide characterized by comprising an amino acid sequence that is the same as or substantially the same as the amino acid sequence represented by SEQ ID NO. 1, or an amide or ester thereof, or a salt thereof;
(2) a polypeptide, or an amide or ester thereof, or a salt thereof according to (1) above, wherein the substantially same amino acid sequence includes the amino acid sequence represented by SEQ ID NO. 11;

(3) a polypeptide, or an amide or ester thereof, or a salt thereof according to (1) above, wherein the substantially same amino acid sequence includes the amino acid sequence represented by SEQ ID NO. 5;
(4) a polypeptide, or an amide or ester thereof, or salt thereof according to (1) above, wherein the substantially same amino acid sequence includes the amino acid sequence represented by SEQ ID NO. 13;
(5) a partial peptide of a polypeptide according to (1) above, or an amide or ester thereof, or salt thereof;
(6) a DNA comprising a DNA coding for the polypeptide according to (1) above;
(7) a DNA according to (6) above, comprising the base sequence represented by SEQ ID NO. 2, 6, 12, or 14;
(8) a DNA comprising DNA coding for the partial peptide according to (5) above;
(9) a recombinant vector comprising the DNA according to (6) or (8) above;
(10) a transformant which is transformed with the recombinant vector according to (9) above;
(11) a method for producing a polypeptide, or an amide or ester thereof, or a salt thereof according to (1) above, or a partial peptide, or an amide or ester thereof, or a salt thereof according to (5) above, characterized by culturing the transformant according to (10) above to allow the production and accumulation of the polypeptide according to (1) above or the partial peptide according to (5) above;
(12) an antibody against a polypeptide, or an amide or ester thereof, or salt thereof according to (1) above, or a partial peptide, or an amide or ester thereof, or salt thereof according to (5) above;
(13) a method for quantifying a polypeptide, or an amide or ester thereof, or salt thereof according to (1) above, or a partial peptide, or an amide or ester thereof, or salt thereof according to (5) above, characterized by the use of the antibody according to (12) above;
(14) a diagnostic agent comprising the DNA according to (6) or (8) above, or the antibody according to (12) above;
(15) an antisense DNA which comprises a base sequence or a portion thereof, complementary or substantially complementary to the base sequence for DNA according to (6) or (8) above, and which has activity capable of inhibiting the expression of the DNA;
(16) an agent comprising a polypeptide, or an amide or ester thereof, or a salt thereof according to (1) above, or a partial peptide, or an amide or ester thereof, or a salt thereof according to (5) above;
(17) a medicinal agent comprising a polypeptide, or an amide or ester thereof, or a salt thereof according to (1) above, or a partial peptide, or an amide or ester thereof, or a salt thereof according to (5) above;
(18) a method for screening a compound or a salt thereof which promotes or inhibits the activity of a polypeptide, or an amide or ester thereof, or a salt thereof according to (1) above, or a partial peptide, or an amide or esters thereof, or a salt thereof according to (5) above, characterized by the use of a polypeptide, or an amide or ester thereof, or a salt thereof according to (1) above, or a partial peptide, or an amide or ester thereof, or a salt thereof according to (5) above;
(19) a screening kit for a compound or a salt thereof which promotes or inhibits the activity of a polypeptide, or an amide or ester thereof, or a salt thereof according to (1) above, or a partial peptide, or an amide or ester thereof, or a salt thereof according to (5) above, comprising a polypeptide, or an amide or ester thereof, or a salt thereof according to (1) above, or a partial peptide, or an amide or ester thereof, or a salt thereof according to (5) above;
(20) a compound or a salt thereof which promotes or inhibits the activity of a polypeptide, or an amide or ester thereof, or a salt thereof according to (1) above, or a partial peptide, or an amide or ester thereof, or a salt thereof according to (5) above, and which is obtained using the screening method according to (18) above or the screening kit according to (19) above; and
(21) a medicinal drug comprising a compound or a salt thereof which promotes or inhibits the activity of a polypeptide, or an amide or ester thereof, or a salt thereof according to (1) above, or a partial peptide, or an amide or ester thereof, or a salt thereof according to (5) above, and which is obtained using the screening method according to (18) above or the screening kit according to (19) above.

The present invention furthermore provides:

(22) a polypeptide, or an amide or ester thereof, or a salt thereof according to (1) above, wherein the amino acid sequence that is substantially the same as the amino acid sequence represented by SEQ ID NO. 1 includes an amino acid sequence having at least about 70%, preferably at least about 80%, more preferably at least about 90%, and even more preferably at least about 95% homology with the amino acid sequence represented by SEQ ID NO. 1; and
(23) a polypeptide, or an amide or ester thereof, or a salt thereof according to (1) above, wherein the amino acid sequence that is substantially the same as the amino acid sequence represented by SEQ ID NO. 1 includes (i) an amino acid sequence in which 1 to 20 (preferably 1 to 15, more preferably 1 to 5, and even more preferably 1 to 3) amino acids are deleted from the amino acid sequence represented by SEQ ID NO. 1; (ii) an amino acid sequence in which 1 to 20 (preferably 1 to 15, more preferably 1 to 5, and even more preferably 1 to 3) amino acids are added to the amino acid sequence represented by SEQ ID NO. 1; (iii) an amino acid sequence in which 1 to 20 (preferably 1 to 15, more preferably 1 to 5, and even more preferably 1 to 3) amino acids are inserted into the amino acid sequence represented by SEQ ID NO. 1; (iv) an amino acid sequence in which 1 to 20 (preferably 1 to 15, more preferably 1 to 5, and even more preferably 1 to 3) amino acids are substituted with other amino acids in the amino acid sequence represented by SEQ ID NO. 1; and (v) an amino acid sequence comprising a combination of the above modifications.

BRIEF DESCRIPTION OF THE DRAWINGS

Figure 1 shows the base sequence of DNA coding for the polypeptide (human type) of the present invention obtained in Example 1;
Figure 2 shows the amino acid sequence of the polypeptide (human type) of the present invention;
Figure 3 compares the amino acid sequence of the polypeptide of the present invention with those of beta subunits of LH, FHS, and TSH;
Figure 4 shows the base sequence of the DNA coding for the polypeptide (rat type) of the present invention obtained in Example 2, and the corresponding amino acid sequence;
Figure 5 compares the sequence of the polypeptide (human type) of the present invention represented by SEQ ID NO. 1 with that of the polypeptide (rat type) of the present invention represented by SEQ ID NO. 11;
Figure 6 shows the result of measuring the antibody titer in antiserum against VH098489, where the antibody titers against GTN1 before (pre-immune serum) and after (antiserum) immunization are compared by the dilution method;
Figure 7 shows the standard curve used in the EIA assay system for VH098499, where the horizontal axis indicates the concentration of the standard (GTN1 peptide), and the vertical axis indicates the binding (B/B0) of the labeled peptide; and
Figure 8 shows the results of analysis by Western blotting for VH098499 expression CHO cells (CHO-GTHL) and control cells (mock), where Western blotting was performed using anti-GTN1 antibodies for both the culture supernatant (sup) and cells (cell), with the molecular weight marker positions indicated on the left of the figure.

BEST MODE FOR CARRYING OUT THE INVENTION

Polypeptides having an amino acid sequence that is the same as or substantially the same as the amino acid sequence represented by SEQ ID NO. 1 (hereinafter sometimes referred to as polypeptides of the present invention) may be synthetic polypeptides, or polypeptides derived from cells (such as retinal cells, liver cells, spleen cells, nerve cells, glia cells, pancreatic β cells, marrow cells, mesangial cells, Langerhans' cells, epidermal cells, epithelial cells, endothelial cells, fibroblasts, fibrocytes, myocytes, adipose cells, immunocytes (such as macrophages, T cells, B cells, natural killer cells, mast cells, neutrophils, basophils, eosinophils, and monocytes), megakaryocytes, synovial cells, chondrocytes, osteocytes, osteoblasts, osteoclasts, mammary gland cells, liver cells, and interstitial cells, or their corresponding precursor cells, stem cells, cancer cells, and the like) or any tissue in which such cells are present, such as the brain, regions of the brain (such as the retina, olfactory bulb, amygdaloid nucleus, basal ganglia, hippocampus, thalamus, hypothalamus, cerebral cortex, medulla oblongata, and cerebellum), spinal cord, pituitary gland, stomach, pancreas, kidneys, liver, gonad, thyroid, gall bladder, bone marrow, adrenal gland, skin, muscle, lungs, gastrointestinal tract (such as the large intestine and small intestine), blood vessels, heart, thymus, spleen, submandibular gland, peripheral blood, prostate gland, testes, ovaries, placenta, uterus, bond, joints, and skeletal muscle, or hematopoietic cells, or cloned cells thereof (such as MEL, M1, CTLL-2, HT-2, WEHI-3, HL-60, JOSK-1, K562, ML-1, MOLT-3, MOLT-4, MOLT-10, CCRF-CEM, TALL-1, Jurkat, CCRT-HSB-2, KE-37, SKW-3, HUT-78, HUT-102, H9, U937, THP-1, HEL, JK-1, CMK, KO-812, and MEG-01) of humans or warm-blooded animals (such as guinea pigs, rats, mice, chickens, rabbits, pigs, sheep, cows, and monkeys).

Examples of amino acid sequences that are substantially the same as the amino acid sequence represented by SEQ ID NO. 1 include amino acid sequences with at least about 70%, preferably at least about 80%, more preferably at least about 90%, and even more preferably at least about 95% homology with the amino acid sequence represented by SEQ ID NO. 1.

Examples of amino acid sequences that are substantially the same as the amino acid sequence represented by SEQ ID NO. 1, other than the above, include the following:

(i) an amino acid sequence wherein 1 to 20, preferably 1 to 15, more preferably 1 to 5, and even more preferably 1 to 3 amino acids are deleted from the amino acid sequence represented by SEQ ID NO. 1 or No. 11;
(ii) an amino acid sequence wherein 1 to 20, preferably 1 to 15, more preferably 1 to 5, and even more preferably 1 to 3 amino acids are added to the amino acid sequence represented by SEQ ID NO. 1 or No. 11;
(iii) an amino acid sequence wherein 1 to 20, preferably 1 to 15, more preferably 1 to 5, and even more preferably 1 to 3 amino acids are inserted into the amino acid sequence represented by SEQ ID NO. 1 or No. 11;
(iv) an amino acid sequence wherein 1 to 20, preferably 1 to 15, more preferably 1 to 5, and even more preferably 1 to 3 amino acids are substituted with other amino acids in the amino acid sequence represented by SEQ ID NO. 1 or No. 11; and
(v) an amino acid sequence comprising a combination of modifications described in (i) through (iv) above;
(vi) an amino acid sequence wherein amino acids(residues) other than Cys at positions 36, 50, 60, 64, 84, 99, 115, 117, 120 and 127 and Asn at position 87 from the N terminal are substituted with other amino acids(residues) in the amino acid sequence represented by SEQ ID NO. 1, or wherein amino acids(residues) other than Cys at positions 35, 49, 59, 63, 83, 98, 114, 116, 119 and 126 and Asn at position 86 from the N terminal are substituted with other amino acids (residues) in the amino acid sequence represented by SEQ ID NO. 11;
(vii) an amino acid sequence wherein 1 to 20 (preferably 1 to 15, more preferably 1 to 5, and even more preferably 1 to 3) amino acids (residues) other than Cys at positions 36, 50, 60, 64, 84, 99, 115, 117, 120 and 127 and Asn at position 87 from the N terminal are substituted with other amino acids (residues) in the amino acid sequence represented by SEQ ID NO. 1, or wherein 1 to 20 (preferably 1 to 15, more preferably 1 to 5, and even more preferably 1 to 3) amino acids (residues) other than Cys at positions 35, 49, 59, 63, 83, 98, 114, 116, 119 and 126 and Asn at position 86 from the N terminal are substituted with other amino acids (residues) in the amino acid sequence represented by SEQ ID No. 11;
(viii) an amino acid sequence wherein the partial amino acid sequence from positions 1 to 24 from the N terminal is deleted from the amino acid sequence reperesented by SEQ ID NO. 1, or wherein the partial amino acid sequence from positions 1 to 23 from the N terminal is deleted from the amino acid sequence represented by SEQ ID NO. 11;
(ix) an amino acid sequence wherein the partial amino acid sequence from positions 1 to 24 from the N terminal is deleted from the amino acid sequence reperesented by SEQ ID NO. 1, or wherein the partial amino acid sequence from positions 1 to 23 from the N terminal is deleted from the amino acid sequence represented by SEQ ID NO. 11; and 1 to 20 (preferably 1 to 15, more preferably 1 to 5, and even more preferably 1 to 3) amino acids are deleted from the remaining amino acid sequence;
(x) an amino acid sequence wherein the partial amino acid sequence from positions 1 to 24 from the N terminal is deleted from the amino acid sequence reperesented by SEQ ID NO. 1, or wherein the partial amino acid sequence from positions 1 to 23 from the N terminal is deleted from the amino acid sequence represented by SEQ ID NO. 11; and 1 to 20 (preferably 1 to 15, more preferably 1 to 5, and even more preferably 1 to 3) amino acids are added to the remaining amino acid sequence;
(xi) an amino acid sequence wherein the partial amino acid sequence from positions 1 to 24 from the N terminal is deleted from the amino acid sequence reperesented by SEQ ID NO. 1, or wherein the partial amino acid sequence from positions 1 to 23 from the N terminal is deleted from the amino acid sequence represented by SEQ ID NO. 11; and 1 to 20 (preferably 1 to 15, more preferably 1 to 5, and even more preferably 1 to 3) amino acids are inserted into the remaining amino acid sequence;
(xii) an amino acid sequence wherein the partial amino acid sequence from positions 1 to 24 from the N terminal is deleted from the amino acid sequence reperesented by SEQ ID NO. 1, or wherein the partial amino acid sequence from positions 1 to 23 from the N terminal is deleted from the amino acid sequence represented by SEQ ID NO. 11; and 1 to 20 (preferably 1 to 15, more preferably 1 to 5, and even more preferably 1 to 3) amino acids are substituted with other amino acids in the remaining amino acid sequence;
(xiii) an amino acid sequence comprising a combination of modifications described in (viii) through (xi) above;
(xiv) an amino acid sequence wherein the partial amino acid sequence from positions 1 to 24 from the N terminal is deleted from the amino acid sequence reperesented by SEQ ID NO. 1, and amino acids other than Cys at positions 36, 50, 60, 64, 84, 99, 115, 117, 120 and 127 and Asn at position 87 from the N terminal are substituted with other amino acids in the remaining amino acid sequence of SEQ ID NO. 1; or wherein the partial amino acid sequence from positions 1 to 23 from the N terminal is deleted from the amino acid sequence represented by SEQ ID NO. 11, and amino acids other than Cys at positions 35, 49, 59, 63, 83, 98, 114, 116, 119 and 126 and Asn at position 86 from the N terminal are substituted with other amino acids (residues) in the remaining amino acid sequence of SEQ ID NO. 11;
(xv) an amino acid sequence wherein the partial amino acid sequence from positions 1 to 24 from the N terminal is deleted from the amino acid sequence reperesented by SEQ ID NO. 1, and 1 to 20 (preferably 1 to 15, more preferably 1 to 5, and even more preferably 1 to 3) amino acids (residues) other than Cys at positions 36, 50, 60, 64, 84, 99, 115, 117, 120 and 127 and Asn at position 87 from the N terminal are substituted with other amino acids in the remaining amino acid sequence of SEQ ID NO. 1; or wherein the partial amino acid sequence from positions 1 to 23 from the N terminal is deleted from the amino acid sequence represented by SEQ ID NO. 11 and 1 to 20 (preferably 1 to 15, more preferably 1 to 5, and even more preferably 1 to 3) amino acids (residues) other than Cys at positions 35, 49, 59, 63, 83, 98, 114, 116, 119 and 126 and Asn at position 86 from the N terminal are substituted with other amino acids in the remaining amino acid sequence of SEQ ID NO. 11;
(xvi) an amino acid sequence wherein 1 to 5, and preferably 1 to 3 amino acids are deleted from the partial amino acid sequence from positions 25 to 36 from the N terminal of the sequence of SEQ ID NO. 1, or deleted from the partial amino acid sequence from positions 24 to 35 from the N terminal of the sequence of SEQ ID NO. 11;
(xvii) an amino acid sequence wherein 1 to 5, and preferably 1 to 3 amino acids are added to the partial amino acid sequence from positions 25 to 36 from the N terminal of the sequence of SEQ ID NO. 1, or added to the partial amino acid sequence from positions 24 to 35 from the N terminal of the sequence of SEQ ID NO. 11;
(xviii) an amino acid sequence wherein 1 to 5, and preferably 1 to 3 amino acids are inserted into the partial amino acid sequence from positions 25 to 36 from the N terminal of the sequence of SEQ ID NO. 1, or inserted to the partial amino acid sequence from positions 24 to 35 from the N terminal of the sequence of SEQ ID NO. 11;
(xix) an amino acid sequence wherein 1 to 5, and preferably 1 to 3 amino acids are substituted with other amino acids in the partial amino acid sequence from positions 25 to 36 from the N terminal of the sequence of SEQ ID NO. 1, or in the partial amino acid sequence from positions 24 to 35 from the N terminal of the sequence of SEQ ID NO. 11; and
(xx) an amino acid sequence comprising a combination of modifications described in (xv) through (xviii) above.

In the present invention, polypeptides having an amino acid sequence substantially the same as the amino acid sequence represented by SEQ ID NO. 1 are preferably polypeptides that have an amino acid sequence substantially the same as the amino acid sequence represented by SEQ ID NO. 1 and that have substantially the same activity as polypeptides having an amino acid sequence substantially the same as the amino acid sequence represented by SEQ ID NO. 1.

Examples of substantially the same activity include the activity of the polypeptides of the present invention (such as activity of preventing and treating the diseases described below, binding activity with a receptor, and cell-stimulating activity on receptor-expressing cells (such as activity in promoting arachidonic acid release, acetylcholine release, intracellular $Ca^{2+}$ release, intracellular cAMP production, intracellular cGMP production, inositol phosphate production, changes in cell membrane potential, intracellular protein phosphorylation, c-fos activation, and decreases in pH)).

"Substantially the same" means that the activity is the same in terms of property (such as physiological or pharmacological one).

Examples of receptors for polypeptides of the present invention include those among a variety of receptors, which have binding activity with polypeptides of the present invention, and through which the polypeptides of the present invention are found to have cell-stimulating activity on cells expressing such receptors (such as activity of promoting arachidonic acid release, acetylcholine release, intracellular $Ca^{2+}$ release, intracellular cAMP production, intracellular cGMP production, inositol phosphate production, changes in cell membrane potential, intracellular protein phosphorylation, c-fos activation, and decreases in pH).

Specific examples include:

(i) LGR4 (AOMF05) (*Molecular Endocrinology* **12**, 1830-1845 (1998); WO99/15545) ;
(ii) LGR5 (HG38) (*Molecular Endocrinology* **12**, 1830-1845 (1998); *BBRC* **247**, 266-270 (1998); and WO99/15660);
(iii) LGR7 (WO99/48921);
(iv) FSH receptors;
(v) LH receptors; and
(vi) TSH receptors.

Specific examples of amino acid sequences which are substantially the same as SEQ ID NO. 1 include the amino acid sequence represented by SEQ ID NO. 5 (that is, an amino acid sequence in which the portion of the amino acid sequence from positions 1 to 24 from the N terminal of the amino acid sequence represented by SEQ ID NO. 1 is deleted), the amino acid sequence represented by SEQ ID NO. 11, and the amino acid sequence represented by SEQ ID NO. 13 (that is, an amino acid sequence in which the portion of the amino acid sequence from positions 1 to 23 from the N terminal of the amino acid sequence represented by SEQ ID NO. 11 is deleted).

In the present Specification, the left end of a peptide is the N terminal (amino terminal) and the right end is the C terminal (carboxyl terminal), according to the usual practice. In the polypeptides of the present invention, including the polypeptide having the amino acid sequence represented by SEQ ID NO. 1, the C-terminal is usually a carboxyl group (-COOH) or a carboxylate ($-COO^-$), but the C-terminal may also be an amide ($-CONH_2$) or an ester (-COOR).

As used herein, R in such esters include $C_1$ to $C_6$ alkyls such as methyl, ethyl, n-propyl, isopropyl, and n-butyl, $C_3$ to $C_8$ cycloalkyls such as cyclopentyl and cyclohexyl, $C_6$ to $C_{12}$ aryls such as phenyl and $\alpha$-naphthyl, phenyl $C_1$ to $C_2$ alkyls such as benzyl or phenethyl, $\alpha$-naphthyl-$C_1$ to $C_2$ alkyls such as $\alpha$-naphthylmethyl, and other such $C_7$

to $C_{14}$ aralkyls, as well as pivaloyloxymethyl groups and the like which are commonly used as esters for oral purposes.

In cases where the polypeptides of the present invention have carboxyl groups (or carboxylates) other than at the C terminal, polypeptides in which such carboxyl groups have been converted to amides or esters are included in the polypeptides of the present invention. Esters in such cases are the same as the C terminal esters and the like described above.

The polypeptides of the present invention include those in which the amino groups of the N terminal amino acid residues (such as methionine residues) are protected with protective groups (such as formyl, acetyl or other such $C_1$ to $C_6$ alkanoyl groups or similar $C_1$ to $C_6$ acyl groups), those in which in vivo cleavage of the N-terminal glutamine residue results in conversion of the Gln to pyroglutamate, those in which substituents (such as -OH, -SH, amino groups, imidazole groups, indole groups, and guanidine groups) on the side chains of the amino acids in the molecule are protected with suitable protective groups (such as formyl, acetyl or other such $C_1$ to $C_6$ alkanoyl groups or similar $C_1$ to $C_6$ acyl groups), and conjugated proteins with what are referred to as glycoproteins comprising sugar linkages.

The sugar chain linkage(attachment) sites in the polypeptides of the present invention can be any to which sugar chains are capable of binding, examples of which include the Asn at position 87 from the N terminal of SEQ ID NO. 1 or the Asn at position 86 from the N terminal of SEQ ID NO. 11.

Examples of constituent sugars for such sugar chains include N-acetyl-D-glucosamine, N-acetyl-D-galactosamine, D-mannose, D-galactose, L-fucose, and sialic acid.

Types of sugar chain linkages include N-glycosidic linkages (bonding between N-acetyl-D-glucosamine and Asn) and O-glycosidic linkages (bonding between N-acetyl-D-galactosamine and Ser or Thr, and bonding between D-galactose and hydroxylysine), although N-glycosidic linkages (bonding between N-acetyl-D-glucosamine and Asn) are the preferred polypeptide linkage in the present invention. Examples of Asn-sugar chain structures with N-glycosidic linkages include high mannose types, complex types, and mixed types.

Specific examples of polypeptides of the present invention include the polypeptide having the amino acid sequence represented by SEQ ID NO. 1, the polypeptide having the amino acid sequence represented by SEQ ID NO. 11, the polypeptide having the amino acid sequence represented by SEQ ID NO. 5, and the polypeptide having the amino acid sequence represented by SEQ ID NO. 13.

Partial peptides of the polypeptides of the present invention may be any partial peptide of the above polypeptides of the present invention.

Specific examples of partial peptides of the polypeptides of the present invention include peptides consisting of the portion of the amino acid sequence from positions 25 to 35 or from positions 121 to 130 from the N terminal of the amino acid sequence represented by SEQ ID NO. 1, and peptides consisting of the portion of the amino acid sequence from positions 24 to 34 or from positions 120 to 129 from the N terminal of the amino acid sequence represented by SEQ ID NO. 11.

Partial peptides of the present invention may also include an amino acid sequence in which 1 to 5 (and preferably 1 to 3) of the amino acids in the above amino acid sequences are deleted, or in which 1 to 5 (and preferably 1 to 3) amino acids are inserted into the above amino acid sequences, or in which 1 to 5 (and preferably 1 to 3) of the amino acids in the above amino acid sequences are substituted with other amino acids, and may also have an amino acid sequence combining any of the above.

In the partial peptides of the present invention, the C-terminal is usually a carboxyl group (-COOH) or a carboxylate ($-COO^-$), but the C-terminal may also be an amide ($-CONH_2$) or an ester (-COOR) (R is the same as defined in the above) in the same manner as polypeptides of the present invention.

In the same manner as polypeptides of the present invention, the partial peptides of the present invention may include those in which the amino groups of the N terminal amino acid residues (such as methionine residues) are protected with protective groups, those in which in vivo cleavage of the N-terminal glutamine residue results in conversion of the glutamine residues to pyroglutamate, and those in which substituents on the side chains of the amino acids in the molecule are protected with suitable protective groups.

Because the partial peptides of the present invention can also be used as antigen to produce antibodies, they need not necessarily have the activity of the polypeptides of the present invention.

Examples of salts of the polypeptides of the present invention or their amides or esters, or of the partial peptides of the present invention or their amides or esters, include pharmaceutically acceptable acids (such as inorganic or organic acids) or bases (such as alkali metal salts), and especially pharmaceutically acceptable acid salts. Examples of such salts include salts of inorganic acids (such as hydrochloric acid, phosphoric acid, hydrobromic acid, and sulfuric acid), and salts of organic acids (such as acetic acid, formic acid, propionic acid, fumaric acid, maleic acid, succinic acid, tartaric acid, citric acid, malic acid, oxalic acid, benzoic acid, methanesulfonic acid, and benzenesulfonic acid).

The polypeptides of the present invention can be produced from the cells or tissue of humans or warm-blooded animals in accordance with a well-known method for polypeptide synthesis. They can also be produced by culturing transformants which have been transformed with DNA coding for polypeptides as described below. Further, the polypeptides of the present invention may be produced in the same way as the peptide synthesis method described later.

When the polypeptides are produced from the cells or tissue of humans or warm-blooded animals, the cells or tissue of humans or warm-blooded animals can be homogenized and then extracted with an acid or the like, and the extract can be purified and isolated by a combination of reverse phase chromatography, ion exchange chromatography, or the like.

Commercially available polypeptide synthesis resins can be used in the synthesis of polypeptides, partial peptides, or their salts or amides in the present invention. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenylacetamidomethyl resin, polyacrylamide resin, 4-(2',4'-dimethoxyphenylhydroxymethyl)phenoxy resin, and 4-(2',4'-dimethoxyphenyl-Fmoc aminoethyl)phenoxy resin. The use of such resins allows amino acids having $\alpha$-amino groups and side chain functional groups protected by suitable protective groups to be condensed on the resin according to the sequence of the target peptide by any of various well-known methods for condensation. After the reaction, the polypeptide can ultimately be cut out of the resin, the various protective groups can be simultaneously removed, and a reaction can be brought about to form intramolecular disulfide bonds in a highly diluted solution, giving the target polypeptide, partial peptide, or amide thereof.

Various activating reagents which can be used for peptide synthesis can be employed in the condensation of the aforementioned protected amino acids, although carbodiimides are particularly preferred. Examples of such carbodiimides include DCC, N,N'-diisopropylcarbodiimide, and N-ethyl-N'-(3-dimethylaminopropyl)carbodiimide. To activate synthesis with such a reagent, a racemization inhibitor additive (such as HOBt or HOOBt) and the protected amino acids can be added to the resin directly, or the inhibitor can be added after the activation of the protected amino acid, in the form of a symmetric acid anhydride or an HOBt ester or HOOBt ester.

Solvents which can be used for the activation of protected amino acids or their condensation with the resin may be selected from known solvents which can be used in peptide condensation. Examples of such solvents include acid amides such as N,N-dimethyl formamide, N,N-dimethyl acetamide, and N-methyl pyrrolidone, halohydrocarbons such as methylene chloride and chloroform, alcohols such as trifluoroethanol, sulfoxides such as dimethyl sulfoxide, ethers such as pyridine, dioxane, and tetrahydrofuran, nitriles such as acetonitrile and propionitrile, esters such as methyl acetate and ethyl acetate, or suitable mixtures thereof. The reaction temperature for the condensation is selected from within the known range known to allow the formation of peptide bonds, which is usually between about -20 °C to 50°C. Activated amino acid derivatives are generally used in a proportion of 1.5- to 4-fold excess. When tests employing a common ninhydrin reaction reveal insufficient condensation, the condensation reaction can be repeated without removing the protective groups until sufficient condensation has been achieved. When repeated condensation fails to result in sufficient condensation, the unreacted amino acids can be acetylated using acetic anhydride or acetylimidazole without affecting subsequent reaction.

Protective groups for the starting material amino acids include Z, Boc, tert-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulfenyl, diphenylphosphinothioyl, and Fmoc.

Carboxyl groups can be protected, for example, by esterification with alkyl esters (such as methyl, ethyl propyl, butyl, t-butyl, cyclopentyl, cyclohexyl, cycloheptyl, cyclooctyl, 2-adamantyl or similar linear, branched, or cyclic alkyl esters), and aralkyl esters (such as benzyl ester, 4-nitrobenzyl ester, 4-methoxybenzyl ester, 4-chlorobenzyl ester, and benzhydryl ester), and phenacyl ester, benzyloxycarbonyl hydrazide, tert-butoxycarbonyl hydrazide, and trityl hydrazide.

Hydroxyl groups of serine can be protected, for example, through esterification or etherification. Groups suitable for such esterification include lower ($C_1$ to $C_6$) alkanoyl groups such as acetyl, aroyl groups such as benzoyl, and carbonic acid-derived groups such as benzyloxycarbonyl and ethoxycarbonyl. Groups suitable for etherification include benzyl, tetrahydropyranyl, and t-butyl.

Protective groups for the phenolic hydroxyl group of tyrosine include Bzl, $Cl_2$-Bzl, 2-nitrobenzyl, Br-Z, and t-butyl.

Examples of protective groups for the imidazole of histidine include Tos, 4-methoxy-2,3,6-trimethylbenzenesulfonyl, DNP, benzyloxymethyl, Bum, Boc, Trt, and Fmoc.

Examples of activated starting material carboxyl groups include the corresponding acid anhydrides, azides, and activated esters (esters of alcohols (such as pentachlorophenol, 2,4,5-trichlorophenol, 2,4-dinitorphenol, cyanomethyl alcohol, para-nitrophenol, HONB, N-hydroxysuccimide, N-hydroxyphthalimide, and HOBt)). Examples of activated starting material amino groups include the corresponding phosphoric amides.

Methods for eliminating (removing) protective groups include catalytic reduction in a hydrogen current in the presence of a catalyst such as Pd black or Pd-carbon, acid treatment with anhydrous hydrogen fluoride, methanesulfonic acid, trifluoromethanesulfonic acid, trifluoroacetic acid or mixtures thereof, treatment with a base such as diisopropylethylamine, triethylamine, piperidine, or piperazine, or reduction with sodium in liquid ammonia. Elimination reactions by the aforementioned acid treatment are generally brought about at a temperature of about -20 to 40°C, but it is effective to add a cation scavenger such as anisole, phenol, thioanisole, meta-cresol, para-cresol, dimethylsulfide, 1,4-butanedithiol or 1,2-ethanedithiol during the acid treatment. The 2,4-dinitrophenyl group used as a protective group for the imidazole of histidine is eliminated by thiophenol treatment, and the formyl group used as the protective group for the indole of tryptophan may be eliminated by acid treatment in the presence of the aforementioned 1,2-ethanedithiol, 1,4-butanedithiol, or the like, and can also be removed by alkali treatment with dilute sodium hydroxide solution, dilute ammonia, or the like.

The protection and deprotection of functional groups which are not involved in the reaction of the starting materials, the elimination of the protective groups, the activation of functional groups involved in the reaction, and the like can be selected from the appropriate known groups and methods.

Another method for obtaining amides of the polypeptides or partial peptides in the present invention is to first protect the α-carboxyl groups of the carboxy terminal amino acids by amidation, to then extend the peptide (polypeptide) chain on the amino group side to the desired length, and to then produce polypeptides in which only the protective groups for the α-amino groups of the N terminal of the peptide chain have been removed and polypeptides in which only the protective groups of the carboxyl groups of the C terminal have been removed in order to condense both polypeptides in the aforementioned solvent mixture. The details of condensation are the same as above. The protected polypeptides obtained by condensation are purified, and all the protective groups can be removed by the aforementioned methods to obtain the desired crude polypeptides. The crude polypeptides can be purified by a number of well known techniques for that purpose, and the primary fractions can be lyophilized to give the desired polypeptide or partial peptide amides.

An example of a way to obtain polypeptide or partial peptide esters of the present invention is to condense the α-carboxyl groups of the carboxy terminal amino acids with a desired alcohol to form an amino acid ester, and to then obtain the desired polypeptide or partial peptide ester in the same manner as polypeptide amides.

Partial peptides or their salts of the present invention can be produced in accordance with a well-known method for peptide synthesis or by cleavage of polypeptides of the present invention using suitable peptidases. The peptides can be synthesized in either the solid or liquid phase, for example. In other words, the target peptide can be produced upon the condensation of a partial polypeptide or amino acid capable of forming a ligand polypeptide with the remainder, and the subsequent elimination of any protective groups when the product has protective groups. The methods in (1) through (5) below are examples of commonly known methods of condensation and methods for eliminating protective groups in such cases.

(1) M. Bodanszky and M. A. Ondetti: *Peptide Synthesis,* Interscience Publishers, New York (1966);
(2) Schroeder and Luebke: *The Peptide,* Academic Press, New York (1965);
(3) Nobuo Izumiya et al.: *Fundamentals and Experiments in Peptide Synthesis,* Maruzen (1975);
(4) Haru'aki Yajima and Shunpei Sakakibara: *Biochemical Experiment Series 1*: *Protein Chemistry IV,* 205 (1977); and
(5) Haru'aki Yajima (ed.), Development of Drugs-Continued, 14, Peptide Synthesis, Hirokawa Shoten.

Following the reaction, the partial peptides of the present invention can be purified and isolated by a combination of common methods of purification such as solvent extraction, distillation, column chromatography, liquid chromatography, and recrystallization. Polypeptides obtained in free form by the above method can be converted to a suitable salt by a common method or a modified method thereof. Conversely, polypeptides obtained in the form of salts can be converted to free form or to another salt by a common method or a modified method thereof.

DNA coding for the polypeptides of the present invention may be any having a base sequence that codes for such polypeptides. Examples include genomic DNA, genomic DNA libraries, cDNA of the aforementioned tissue and cells, cDNA libraries of such tissue and cells, and synthetic DNA.

Vectors used in libraries may be any from among bacteriophages, plasmids, cosmids, phagemids, and the like. Total RNA and mRNA fractions prepared from such tissue and cells can also be amplified by reverse transcriptase polymerase chain reaction (RT-PCR).

Examples of DNA coding for polypeptides of the present invention include DNA having the base sequence represented by SEQ ID NO. 2 or 12, or DNA which has a base sequence hybridizing under highly stringent conditions with the base sequence represented by SEQ ID NO. 2 or 12, and which codes for a polypeptide having substantially the same activity as polypeptides of the present invention.

Examples of DNA capable of hybridizing under highly stringent conditions with the base sequence represented by SEQ ID NO. 2 or 12 include DNA containing a base sequence with at least about 70%, preferably at least about 80%, more preferably at least 90%, and even more preferably at least 95% homology with the base sequence represented by SEQ ID NO. 2.

Hybridization can be carried out in accordance with a well-known method or a modified method thereof, such as the methods given in *Molecular* Cloning 2$^{nd}$ Ed. (J. Sambrook et al., Cold Spring Harbor Lab. Press (1989)). When a commercially available DNA library is used, the method given in the accompanying protocol should be followed.

Hybridization is more preferably capable of being carried out under highly stringent conditions.

Highly stringent conditions refer to conditions involving, for example, a sodium concentration of about 19 to 40 mM and preferably about 19 to 20 mM, and a temperature of about 50 to 70°C, and preferably about 60 to 65°C. A sodium concentration of about 19 mM and a temperature of about 65°C are most preferable.

Examples of DNA coding for polypeptides having activity substantially the same as the polypeptides of the present invention include DNA having the base sequence represented by SEQ ID NO. 6 or 14.

More specific examples of DNA coding for the polypeptide having the amino acid sequence represented by SEQ ID NO. 1 include DNA having the base sequence represented by SEQ ID NO. 2. Examples of DNA coding for the polypeptide having the amino acid sequence represented by SEQ ID NO. 5 include DNA having the base sequence represented by SEQ ID NO. 6. Examples of DNA coding for the polypeptide having the amino acid sequence represented by SEQ ID NO. 11 include DNA having the base sequence represented by SEQ ID NO. 12. Examples of DNA coding for the polypeptide having the amino acid sequence represented by SEQ ID NO. 13 include DNA having the base sequence represented by SEQ ID NO. 14.

Examples of DNA coding for partial peptides of the present invention include any having a base sequence coding for such partial peptides of the present invention. Examples include genomic DNA, genomic DNA libraries, cDNA of the aforementioned tissue and cells, cDNA libraries of such tissue and cells, and synthetic DNA.

Examples of DNA coding for partial peptides of the present invention include DNA having a portion of the base sequence of DNA having the base sequence represented by SEQ ID NO. 2 or 12, or DNA which has a base sequence hybridizing under highly stringent conditions with the base sequence represented by SEQ ID NO. 2 or 12, and which has a portion of the base sequence of DNA coding for a polypeptide having substantially the same activity as polypeptides of the present invention.

DNA capable of hybridizing with the base sequence of represented by SEQ ID NO. 2 or 12 is defined the same as above.

Methods of hybridization and highly stringent conditions are also the same as above.

Polypeptides encoded by DNA capable of hybridizing with the base sequence represented by SEQ ID NO. 2 or 12 can be produced in the same manner as the methods described below for the production of polypeptides of the present invention. Amides, esters, and salts of such polypeptides are the same as amides, esters, and salts of polypeptides of the present invention described above.

More specific examples of DNA coding for partial peptides of the present invention include DNA containing DNA having a base sequence coding for a peptide consisting of the portion of the amino acid sequence from positions 25 to 35 from the N terminal in the amino acid sequence represented by SEQ ID NO. 1 or the portion of the amino acid sequence from positions 24 to 34 from the N terminal in the amino acid sequence represented by SEQ ID NO. 11, or a peptide consisting of the portion of the amino acid sequence from positions 121 to 130 from the N terminal in the amino acid sequence represented by SEQ ID NO. 1 or the portion of the amino acid sequence from positions 121 to 130 from the N terminal in the amino acid sequence represented by SEQ ID NO. 11; or DNA containing DNA having a base sequence hybridizing under highly stringent conditions with the above.

The polypeptides or partial peptides of the present invention, or DNA coding for the polypeptides or partial peptides of the present invention, may be labeled by a well-known method. Specific examples include those that are isotope-labeled, fluorescent-labeled (such as with fluoroscein), biotin-labeled, or enzyme-labeled.

Procedures for cloning DNA fully coding for the polypeptides or partial peptides of the present invention (such polypeptides and the like are sometimes simply referred to below as polypeptides of the present invention when describing the cloning and expression of DNA coding for such polypeptides and the like) include amplification of DNA by the well-known PCR method using synthetic DNA primers having a portion of the base sequence coding for polypeptides of the invention, or selection of DNA contained in suitable vectors by hybridization with labeled synthetic DNA or DNA fragments coding for some or all regions of polypeptides of the present invention. Hybridization should be carried out in accordance with the methods given, for example, in *Molecular Cloning* 2nd Ed. (J. Sambrook et al., Cold Spring Harbor Lab. Press (1989)). When a commercially available DNA library is used, the method given in the accompanying protocol should be followed.

The DNA base sequence can be changed by a well-known method or a modified method thereof, such as ODA-LA PCR, the gapped duplex method, or the Kunkel method, using PCR or a common kit such as Mutan™-Super Express Km (Takara Shuzo) or Mutan™-K (Takara Shuzo).

The cloned DNA coding for the polypeptide can be used as such or after being digested with the desired restriction enzymes or after the addition of linker DNA, depending on the intended purpose. The DNA may have ATG as the translation initiation codon on the 5' terminal side, and TAA, TGA, or TAG as the translation termination codon on the 3' terminal side. The translation initiation and termination codons can be added using suitable synthetic DNA adapters.

Expression vectors for the polypeptides of the present invention can be prepared, for example, by

(a) cutting out the target DNA fragment from DNA coding for a polypeptide of the present invention, and
(b) ligating the DNA fragment downstream of a promoter in a suitable expression vector.

Examples of vectors which can be used include *E. coli* plasmids (such as pBR322, pBR325, pUC12, and pUC13), *Bacillus subtilis* plasmids (such as PUB110, pTP5, and pC194), yeast plasmids (such as pSH19 and pSH15), bacteriophages such as λ phages, and animal viruses such as retroviruses, vaccinia viruses, and baculoviruses, as well as pA1-11, pXT1, pRc/CMV, pRc/RSV, and pcDNA I/Neo.
Promoters that can be used in the present invention include any that are suitable for the host which is used to express the gene. Examples for animal cell hosts include SRα promoters, SV40 promoters, HIV-LTR promoters, CMV promoters, and HSV-TK promoters.
CMV (cytomegalovirus) promoters and SRα promoters are preferred among these. Promoters that are preferred for *E. coli* hosts include trp promoters, lac promoters, recA promoters, λPL promoters, lpp promoters, and T7 promoters; examples that are preferred for Bacillus hosts include SPO1 promoters, SPO2 promoters, and penP promoters; and examples that are preferred for yeast hosts include PHO5 promoters, PGK promoters, GAP promoters, and ADH promoters. Examples that are preferred for insect cell hosts include polyhedrin and P10 promoters.
In addition to the above, expression vectors can also contain enhancers, splicing signals, poly A linker signals, selection markers, SV40 replication origins (sometimes referred to below as SV40ori), and the like as needed. Examples of selection markers include the dihydrofolate reductase (sometimes referred to below simply as dhfr) gene {methotrexate (MTX) resistance}, ampicillin resistance gene (sometimes referred to below simply as $Amp^r$), and neomycin resistance gene (G418 resistance, sometimes referred to below simply as $Neo^r$). In particular, the target gene can also be selected with thymidine-free medium when the dhfr gene is used as the selection marker with DHFR-deficient Chinese hamster ovary cells.
A signal sequence compatible with the host can be attached to the N-terminal side of polypeptides of the present invention if needed. Examples of signal sequences for Escherichia hosts include PhoA signal sequences and OmpA signal sequences; examples for Bacillus hosts include α-amylase signal sequences and subtilisin signal sequences; examples for yeast hosts include MFα signal sequences and SUC2 signal sequences; and examples for animal cell hosts include insulin signal sequences, α-interferon signal sequences, and antibody molecule signal sequences.
The resulting vectors containing DNA coding for polypeptides of the present invention can be used to produce transformants.
Examples of hosts that can be used include Escherichia microorganisms, Bacillus microorganisms, yeasts, insect cells, insects, and animal cells.
Specific examples of Escherichia microorganisms include *Escherichia coli* K12·DH1 (*Proc. Natl. Acad. Sci. USA,* **60**, 160 (1968)), JM103 (*Nucleic Acids Research*, **9**, 309 (1981)), JA221 (*Journal of Molecular Biology*, **120**, 517 (1978)), HB101 (*Journal of Molecular Biology,* **41**, 459 (1969)), and C600 (*Genetics,* **39**, 440 (1954)).
Examples of Bacillus microorganisms include *Bacillus subtilis* MI114 (*Gene,* **24**, 255 (1983)) and 207-21 (*Journal of Biochemistry,* **95**, 87 (1984)).
Examples of yeasts include *Saccharomyces cerevisiae* AH22, $AH22R^-$, NA87-11A, DKD-5D, 20B-12, *Schizosaccharomyces pombe* NCYC1913 and NCYC2036, and *Pichia pastoris* KM71.
Examples of insect cells for the AcNPV virus include established cell lines from the larvae of *Spodoptera frugiperda cell* (Sf cells), MG1 cells from the interior lining of the gut of *Trichoplusia ni,* High Five™ cells from *Trichoplusia ni* ova, *Mamestra brassicae* cells, and *Estigmena acrea* cells. Examples for the BmNPV virus include the *Bombyx mori* cell line (*Bombyx mori* N cells; BmN cells). Examples of Sf cells include Sf9 cells (ATCC CRL 1711) and Sf21 cells (both by J.L. Vaughn et al., *In Vivo,* **13**, 213-217(1977)).
Examples of insects include silkworm larvae (Maeda et al, *Nature,* **315**, 592 (1985)).
Examples of animal cells include monkey cells COS-7, Vero, Chinese hamster ovary cells (CHO cells), DHFR-deficient Chinese hamster ovary cells (CHO ($dhfr^-$) cells), mouse L cells, mouse AtT-20, mouse myeloma cells, rat GH3, and human FL cells.
Escherichia microorganisms can be transformed in accordance with methods as disclosed in, for example, *Proc. Natl. Acad. Sci. USA,* **69**, 2110 (1972), and *Gene,* **17**, 107 (1982).
Bacillus microorganisms can be transformed in accordance with methods as disclosed in, for example, *Molecular & General Genetics,* **168**, 111 (1979).
Yeasts can be transformed in accordance with methods as disclosed in, for example, *Methods in Enzymology,* **194**, 182-187 (1991), and *Proc. Natl. Acad. Sci. USA,* **75**, 1929 (1978).
Insect cells or insects can be transformed in accordance with methods as disclosed in, for example, *Bio/ Technology*, **6**, 47-55 (1988).
Animal cells can be transformed by methods as disclosed in, for example, *Saibo Kogaku [Cell Engineering]* Special Edition No. 8: *Shin Saibo Kogaku Jikken Purotokoru [New Cell Engineering Experimental Protocols],* 263-267, published by Shujunsha (1995), and *Virology,* **52**, 456 (1973).

In this manner it is possible to obtain transformants which have been transformed with expression vectors that contain DNA coding for polypeptides.

Liquid media are preferred for the culture of Escherichia or Bacillus transformants, and should be prepared with the carbon sources, nitrogen sources, minerals, and the like which are needed for the growth of the transformants. Examples of carbon sources include glucose, dextrin, soluble starch, and sucrose. Examples of nitrogen sources include organic or inorganic substances such as ammonium salts, nitrates, corn steep liquor, peptone, casein, meat extract, soybean cake, and potato extract. Examples of minerals include calcium chloride, sodium dihydrogen phosphate, and magnesium chloride. Yeast extract, vitamins, growth-promoting factors, and the like may also be added. The medium pH is preferably about 5 to 8.

The preferred medium for culturing Escherichia microorganisms is M9 medium containing, for example, glucose and casamino acid (Miller, *Journal of Experiments in Molecular Genetics,* pp. 431-433, Cold Spring Harbor Laboratory, New York (1972)). The medium may be supplemented as needed with agents such as 3β-indolyl acrylic acid in order to ensure promoter efficiency.

Escherichia transformants can usually be cultured for about 3 to 24 hours at about 15 to 43°C, while stirred and aerated as needed.

Bacillus transformants can usually be cultured for about 6 to 24 hours at about 30 to 40°C, while aerated or stirred as needed.

Examples of media for the culture of transformants of yeast include Burkholder minimum medium (K.L. Bostian et al., *Proc. Natl. Acad. Sci. USA,* **77**, 4505 (1980)), and SD medium containing 0.5% casamino acid (G.A. Bitter et al., *Proc. Natl. Acad. Sci. USA,* **81**, 5330 (1984)). The medium pH should be adjusted to between about 5 and 8. Culture usually lasts about 24 to 72 hours at about 20 to 35°C, while aerated and stirred as needed.

Examples of media for the culture of transformants of insect cell hosts or insect hosts include Grace's insect medium (T.C.C. Grace, *Nature,* **195**, 788 (1962)) suitably supplemented with an additive such as 10% inactivated bovine serum. The medium pH should be adjusted to between about 6.2 and 6.4. Culture usually lasts about 3 to 5 days at about 27°C, while aerated and stirred as needed.

Examples of media for the culture of transformants of animal cell hosts include MEM medium supplemented with about 5 to 20% fetal calf serum *(Science,* **122**, 501 (1952)), DMEM medium (*Virology,* **8**, 396 (1959)), RPMI 1640 medium (*Journal of the American Medical Association,* **199**, 519 (1967)), and 199 medium (Proceedings of the Society for the Biological Medicines, **73**, 1 (1950)). The pH should be between about 6 and 8. Culture usually lasts about 15 to 60 hours at about 30 to 40°C, while aerated and stirred as needed.

In this manner, it is possible to produce polypeptides of the present invention in cell membranes and the like of the transformants.

Polypeptides of the present invention can be isolated and purified from the above cultures in the following manner.

When polypeptides of the invention are extracted from cultured bacteria or cells, the bacteria or cells are collected in the usual manner after the culture and are suspended in a suitable buffer, the cells are then disrupted by common ultrasonic treatment, lysozyme treatment, and/or freeze-thawing or the like, and a crude extract of the polypeptides is then obtained by common centrifugation, filtration, or the like. The buffer may be supplemented with protein denaturants such as urea or guanidine hydrochloride, or surfactant such as Triton X-100™. When the polypeptides are secreted in the culture, the cultured bacterial cells or cultured cells are separated in the usual manner from the supernatant, giving polypeptides in the form of culture supernatant.

The polypeptides contained in the resulting culture supernatant or extract can be purified by a suitable combination of well-known isolation and purification methods. Examples of such methods include: methods exploiting solubility, such as salting out or solvent precipitation; methods primarily exploiting differences in molecular weight, such as dialysis, ultrafiltration, gel filtration and SDS-polyacrylamide gel electrophoresis; methods exploiting differences in electrical charge, such as ion-exchange chromatography; methods exploiting specific affinity, such as affinity chromatography; methods exploiting differences in hydrophobicity, such as reverse-phase high-performance liquid chromatography; and methods exploiting differences in isoelectric point, such as isoelectric focusing.

Polypeptides obtained in free form can be converted to a salt by a well-known method or a modified method thereof. Conversely, polypeptides obtained in the form of salts can be converted to free form or to another salt by a well-known method or a modified method thereof.

Either before or after the purification of a polypeptide produced with recombinants, a suitable protein-modifying enzyme can be allowed to act thereon in the usual manner to add any modifications or to remove portions of the polypeptide. Examples of such enzymes include trypsin, chymotrypsin, arginyl endopeptidase, protein kinase, and glycosidase.

Antibodies against polypeptides and partial peptides of the invention, or their esters or amides, or salts thereof, may be any polyclonal or monoclonal antibody capable of recognizing antibodies against the polypeptides and partial peptides of the invention, or their esters or amides, or salts thereof.

Antibodies against polypeptides and partial peptides of the invention, or their esters or amides, or salts thereof (in the following description of antibodies, these are sometimes referred to simply as polypeptides of the present invention) may be produced by using polypeptides of the invention as antigen according to a well-known method for producing antibodies and antiserum.

[Preparation of Monoclonal Antibodies]

(a) Preparation of Monoclonal Antibody-Producing Cells

Polypeptides of the present invention are administered, either alone or along with a carrier and diluent, to warm-blooded animals at a site permitting the production of antibodies. Freund's complete adjuvant or incomplete adjuvant may also be given in order to potentiate the production of antibodies during administration. Administration is usually once every 2 to 6 weeks, for a total of about 2 to 10 times. Warm-blooded animals that can be used include monkeys, rabbits, dogs, guinea pigs, mice, rats, sheep, goats and chickens. The use of mice and rats is preferred, although mice and rats are preferred.

In the preparation of cells which produce monoclonal antibodies, individual animals with suitable antibody titer can be selected from animals such as mice which have been immunized with antigen, the spleens or lymph nodes can be harvested 2 to 5 days after final immunization, and the antibody-producing cells obtained therefrom can be fused with myeloma cells from animals of the same or different species so as to prepare monoclonal antibody-producing hybridomas. The antibody titer in antiserum can be assayed, for example, by bringing about a reaction between antiserum and labeled protein as described below, and by then assaying the activity of the labeled material bound to the antibody. Fusion can be carried out, for example, in accordance with the method of Koehler and Milstein (*Nature,* **256**, 495, (1975)). Examples of fusion promoters include polyethylene glycol (PEG) and the Sendai virus, although the use of PEG is preferred.

Examples of myeloma cells include those of warm-blooded animals, such as NS-1, P3U1, SP2/0, and AP-1, although the use of P3U1 is preferred. The proportion between the number of antibody-producing cells (spleen cells) and the number of myeloma cells is preferably about 1:1 to 20:1. Efficient cell fusion can be achieved by 1 to 10 minutes of incubation at 20 to 40°C, and preferably 30 to 37°C, with the addition of PEG (preferably, PEG 1000 to PEG 6000) in a concentration of about 10 to 80%

A variety of methods can be employed to screen monoclonal antibody-producing hybridomas, such as methods in which hybridoma culture supernatant is added to a solid phase (such as a microplate) to which the polypeptide (protein) antigen is adsorbed, either directly or with a carrier, and protein A or anti-immunoglobulin antibody (anti-mouse immunoglobulin antibody when the cells used for cell fusion are from a mouse) labeled with a radioactive substance, an enzyme, or the like is added to detect monoclonal antibodies binding to the solid phase; or methods in which hybridoma supernatant liquid is added to a solid phase to which anti-immunoglobulin or protein A is adsorbed, polypeptides labeled with a radioactive substance, an enzyme, or the like are added, and monoclonal antibodies binding to the solid phase are detected.

Monoclonal antibodies can be selected according to a well-known method or a modified method thereof. This can usually be done in animal cell media containing HAT (hypoxanthine, aminopterin, and thymidine). Any medium may be used as long as hybridomas are able to grow therein. Any medium allowing hybridomas to grow can be used for selection and growth. Examples include RPMI 1640 medium containing 1 to 20%, and preferably 10 to 20%, fetal calf serum, GIT medium (Wako Pure Chemicals) containing 1 to 10% fetal calf serum, and serum-free medium (SFM-101, by Nissui Seiyaku) for hybridoma culture. The culture temperature is usually 20 to 40°C, and preferably about 37°C. The culture usually lasts from 5 days to 3 weeks, and preferably 1 to 2 weeks. The culture can usually take place with 5% carbon dioxide gas. The antibody titer of the hybridoma culture supernatant can be assayed in the same manner as in the aforementioned assay of the antibody titer in antiserum.

(b) Purification of Monoclonal Antibodies

The monoclonal antibodies can be isolated and purified by common methods such as methods for isolating and purifying immunoglobulin (for example, salting-out, precipitation with alcohol, isoelectric precipitation, electrophoresis, adsorption and desorption using ion exchangers (such as DEAE), ultracentrifugation, gel filtration, and specific methods of purification in which only antibodies are collected using an active adsorbent such as an antigen-binding solid phase, protein A, or protein G, and antibodies are obtained upon the dissociation of the bonds).

[Production of Polyclonal Antibodies]

Polyclonal antibodies of the present invention can be produced by a well-known method or a modified method thereof. For example, immunogen (polypeptide antigen) itself or in the form of a complex with a carrier protein can be prepared, warm-blooded animals can be immunized in the same manner as in the production of monoclonal antibodies, materials containing antibody against polypeptides of the invention can be harvested from the immunized animals, and the antibodies can be isolated and purified.

Any type of carrier protein can be mixed in any proportion relative to hapten in the immunogen-carrier protein conjugates used to immunize warm-blooded animals, as long as they result in the efficient production of antibodies against hapten when crosslinked with the carrier for immunization. For example, bovine serum albumin, bovine thyroglobulin, or hemocyanin can be coupled in a weight ratio of about 0.1 to 20, and preferably about 1 to about 5, per unit hapten.

Various condensation agents can be used to couple the hapten and carrier. Glutaraldehyde, carbodiimides, maleimide active esters, and active ester reagents with thiol and dithiopyridyl groups can be used.

The condensation reaction product is administered, either alone or along with a carrier and diluent, to warm-blooded animals at a site permitting the production of antibodies. Freund's complete adjuvant or incomplete adjuvant may also be given in order to potentiate the production of antibodies during administration. Administration is usually once every 2 to 6 weeks, for a total of about 3 to 10 times.

Polyclonal antibodies can be harvested from the blood, ascites fluid, or the like of warm-blooded animals immunized in the manner described above, and are preferably harvested from the blood.

The titer of the polyclonal antibody in antiserum can be assayed in the same manner as in the assay of the antibody titer in antiserum described above, The polyclonal antibodies can be isolated and purified in accordance with methods for the isolation and purification of immunoglobulin in the same manner as the isolation and purification of the aforementioned monoclonal antibodies.

Examples of antisense DNA having a base sequence or a portion thereof which is complementary to, or substantially complementary to the base sequence of DNA coding for polypeptides or partial peptides of the present invention (referred to as DNA of the invention in the description of antisense DNA below) include any having a base sequence or a portion thereof which is complementary to, or substantially complementary to the base sequence of DNA of the present invention, provided that it has activity of suppressing the expression of such DNA.

Examples of base sequences or partial sequences thereof that are substantially complementary to base sequences of DNA of the present invention include base sequences with at least about 70%, preferably at least about 80%, more preferably at least about 90%, and even more preferably at least about 95% homology with all or part of a base sequence complementary to a base sequence of DNA of the present invention (that is, complementary sequences of DNA of the present invention). Among these complementary sequences of DNA of the present invention, preferred is antisense DNA with at least about 70%, preferably at least about 80%, more preferably at least about 90%, and even more preferably at least about 95% homology with complementary sequences of partial base sequences coding for the N-terminal regions (such as base sequences near the initiation codon) of polypeptides of the present invention. Such antisense DNA can be produced using a common DNA synthesizer, for example.

The following descriptions relate to applications of (i) the polypeptide, or its amide or ester, or salt thereof, or the partial peptide, or its amide or ester, or salt thereof of the present invention (sometimes referred to below simply as the polypeptide of the invention); (ii) DNA coding for the polypeptide or partial peptide of the invention, or DNA coding for the receptor protein or partial peptide thereof of the invention (sometimes referred to below simply as DNA of the invention); (iii) the antibody against the polypeptide of the invention (sometimes referred to below simply as the antibody of the invention); and (iv) the antisense DNA.

[1] Agents for the treatment and prevention of various diseases in which polypeptides of the invention are involved

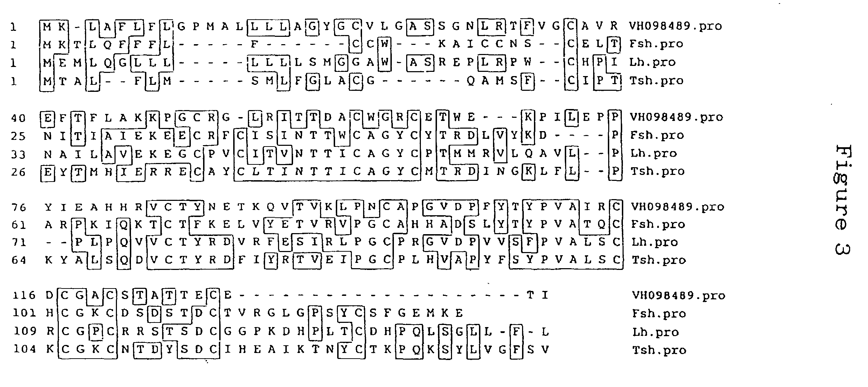

As demonstrated in Example 1 and Figure 3 below, the polypeptides of the invention show high homology with the beta subunits known for LH, FSH, and TSH, while their association with other subunits (such as alpha subunits, specifically, polypeptides having an amino acid sequence that is the same as or substantially the same as the amino acid sequence represented by SEQ ID NO. 7; "substantially the same" means that the activity of the alpha subunits or the activity manifested upon the association of polypeptides of the present invention with alpha subunits is the same in terms of property (such as physiological or pharmacological one), in the same manner as described above for polypeptides of the present invention) results in the manifestation of their physiological activity.

The polypeptides of the present invention are expected to have disulfide bonds, in the same manner as known LH, FSH, TSH, and the like, between positions 36 (Cys) and 84 (Cys), positions 50 (Cys) and 99 (Cys), positions 60 (Cys) and 115 (Cys), positions 64 (Cys) and 117 (Cys), and positions 120 (Cys) and 127 (Cys) in SEQ ID NO. 1, and between positions 35 (Cys) and 83 (Cys), positions 49 (Cys) and 98 (Cys), positions 59 (Cys) and 114 (Cys), positions 63 (Cys) and 116 (Cys), and positions 119 (Cys) and 126 (Cys) in SEQ ID NO. 11. As noted in Example 1 below, the polypeptides of the present invention show the physiological activity in itself without associating with other subunits, due to a lack of Cys capable of disulfide bonds, which is considered a necessary factor for association with alpha subunits in known LH, FSH, TSH, and the like.

Thus, aberration or deletion of DNA coding for the polypeptides of the invention, or DNA coding for receptor proteins of the polypeptides of the invention, may result in the high possibility of diseases such as hypertension, autoimmune diseases, cardiac failure, cataracts, glaucoma, acute bacterial meningitis, acute myocardial infarction, acute pancreatitis, acute viral encephalitis, adult respiratory distress syndrome, alcoholic hepatitis, Alzheimer's disease, asthma, atherosclerosis, atopic dermatitis, bacterial pneumonia, bladder carcinoma, bone fractures, breast cancer, hyperexia, adphagia, burn treatment, uterine cancer, chronic lymphatic leukemia, chronic myeloid leukemia, chronic pancreatitis, hepatic cirrhosis, colon cancer (colorectal cancer), Crohn's disease, dementia, diabetic complications, diabetic nephropathy, diabetic neuropathy, diabetic retinopathy, gastritis, *Helicobacter pylori* infection, hepatic insufficiency, hepatitis A, hepatitis B, hepatitis C, hepatitis, Herpes simplex, Herpes zoster, Hodgkin's disease, AIDS, human papilloma viral infections, hypercalcemia, hypercholesterolemia, hypertriglyceridemia, hyperlipemia, infections, influenza viral infections, insulin-dependent diabetes (type I), invasive Staphylococcus infection, malignant melanoma, metastatic cancer, multiple myeloma, allergic rhinitis, nephritis, non-Hodgkin's lymphoma, non-insulin-dependent diabetes (type II), non-small cell lung cancer, organ transplants, osteoarthropy, osteomalacia, osteopenia, osteoporosis, ovarian cancer, bone Paget disease, peptic ulcers, peripheral blood vessel disease, prostate cancer, esophageal reflux, renal insufficiency, rheumatoid arthritis, schizophrenia, sepsis, septic shock, serious systemic mycosis, small-cell lung cancer, spinal injuries, stomach cancer, systemic lupus erythematosus, transient ischemic attack, tuberculosis, valvular heart disease, vascular/multi-infarct dementia, trauma therapy, insomnia, arthritis, insufficiency of pituitary hormone secretion, pollakiuria, uremia, and neurodegenerative diseases.

The polypeptides of the present invention and the DNA of the present invention can therefore be used as drugs for the prevention and treatment of such diseases.

The polypeptides and DNA of the present invention are useful, for example, in patients with no or lower level of the endogenous polypeptide of the present invention, by (a) administration of DNA of the present invention to the patients to bring about the in vivo expression of polypeptides of the invention, (b) incorporation of DNA of the present invention into cells to express polypeptides of the invention, and subsequent implantation of the cells to the patients, or (c) administration of polypeptides of the present invention to the patients, so as to ensure sufficient or normal function of the polypeptides of the invention in such patients.

When DNA of the present invention is used as a therapeutic and prophylactic agent as described above, the DNA can be administered, either alone or after being inserted into a suitable vectors such as a retrovirus vector, adenovirus vector, or adenovirus-associatedvirus vector, to humans or warm-blooded animals in the usual manner. DNA of the invention can be administered, either as such or in the form of a preparation containing a physiologically acceptable carrier such as an adjuvant to facilitate ingestion, by means of a gene gun or a catheter such as a hydrogel catheter.

When polypeptides of the present invention are used for the therapeutic and prophylactic purposes described above, they should be purified to at least 90%, preferably at least 95%, more preferably at least 98%, and even more preferably at least 99%.

The polypeptides of the present invention can be used, for example, orally in the form of optionally sugar-coated tablets, capsules, elixirs, microcapsules or the like, or they can be used parenterally in the form of injections such as sterile solutions or suspensions with water or other pharmaceutically acceptable liquids. These preparations can be produced, for example, by mixing a polypeptide of the invention with physiologically acceptable carriers, flavoring agents, excipients, vehicles, antiseptics, stabilizers, binders, or the like, in the unit dose forms required in generally accepted pharmaceutical manufacturing. The content of the active ingredient in these preparations should give the appropriate dose within the specified range.

Examples of additives which can be mixed with tablets, capsules, and the like include binders such as gelatin, corn starch, tragacanth, and gum arabic; excipients such as crystalline cellulose; extenders such as corn starch, gelatin, and alginic acid; lubricants such as magnesium stearate; sweetening agents such as sucrose, lactose, and saccharin; and flavoring agents such as peppermint, akamono oil, and cherry. In the case of capsule unit dose forms, the aforementioned types of materials can also include liquid carriers such as oils and fats. Sterile compositions for injection can be formulated by ordinary pharmaceutical manufacturing methods such as the dissolution or suspension of active ingredients and naturally occurring vegetable oils such as sesame oil or coconut oil in a vehicle such as water for injection.

Aqueous liquids for injection include physiological saline and isotonic solutions containing glucose or other adjuvants (such as D-sorbitol, D-mannitol, and sodium chloride), and may be used in combination with appropriate dissolution aids such as alcohols (such as ethanol), polyalcohols (such as propylene glycol and polyethylene glycol), and nonionic surfactants (such as Polysorbate 80™ and HCO-50). Oleaginous liquids include sesame oil and soybean oil, and may be used in combination with dissolution aids such as benzyl benzoate and benzyl alcohol. The above may also be blended with buffers (such as phosphate buffer and sodium acetate buffer), soothing agents (such as benzalkonium chloride and procaine hydrochloride), stabilizers (such as human serum albumin and polyethylene glycol), preservatives (such as benzyl alcohol and phenol), antioxidants, and the like. Suitable ampules are usually aseptically filled with the resulting injection liquid.

Vectors containing the DNA of the invention may also be similarly formulated, and are usually used parenterally.

Because such preparations are safe and have low toxicity, they can be administered, for example, to humans and warm-blooded animals (such as rats, mice, guinea pigs, rabbits, birds, sheep, pigs, cows, horses, cats, dogs, and monkeys).

The dosage of polypeptides of the present invention will vary depending on the target disease, purpose of administration, route of administration or the like. For example, for the treatment of neurological diseases, the daily oral dosage of polypeptides of the invention for adults may generally range from about 0.1 to 100 mg, preferably from about 1.0 to 50 mg, and even more preferably from about 1.0 to 20 mg(per 60 kg body weight). The single parenteral dose of polypeptides will vary depending on the purpose of administration, target disease, and the like. For example, for treatment of neurological diseases in the form of an injection for adults, the daily dosage of polypeptides of the invention may usually range from about 0.01 to about 30 mg, preferably about 0.1 to about 20 mg, and even more preferably about 0.1 to about 10 mg at a time (per 60 kg body weight), given by injection to the affected site. Doses for animals can be given as calculated per 60 kg body weight.

[2] Screening of Candidate Therapeutic Compounds for Diseases

The polypeptides of the present invention have physiological activity related to anterior pituitary hormones (such as LH, FSH, and TSH), and compounds or their salts which promote or inhibit the functions of the polypeptides of the present invention (such as physiological activity related to anterior pituitary hormones (such as LH, FSH, and TSH)) can thus be used as drugs for the treatment and prevention of various diseases such as hypertension, autoimmune diseases, cardiac failure, cataracts, glaucoma, acute bacterial meningitis, acute myocardial infarction, acute pancreatitis, acute viral encephalitis, adult respiratory distress syndrome, alcoholic hepatitis, Alzheimer's disease, asthma, atherosclerosis, atopic dermatitis, bacterial pneumonia, bladder carcinoma, bone fractures, breast cancer, hyperexia, adphagia, burn treatment, uterine cancer, chronic lymphatic leukemia, chronic myeloid leukemia, chronic pancreatitis, hepatic cirrhosis, colon cancer (colorectal cancer), Crohn's disease, dementia, diabetic complications, diabetic nephropathy, diabetic neuropathy, diabetic retinopathy, gastritis, *Helicobacter pylori* infection, hepatic insufficiency, hepatitis A, hepatitis B, hepatitis C, hepatitis, Herpes simplex, Herpes zoster, Hodgkin's disease, AIDS, human papilloma viral infections, hypercalcemia, hypercholesterolemia, hypertriglyceridemia, hyperlipemia, infections, influenza viral infections, insulin-dependent diabetes (type I), invasive Staphylococcus infection, malignant melanoma, metastatic cancer, multiple myeloma, allergic rhinitis, nephritis, non-Hodgkin's lymphoma, non-insulin-dependent diabetes (type II), non-small cell lung cancer, organ transplants, osteoarthropy, osteomalacia, osteopenia, osteoporosis, ovarian cancer, bone Paget disease, peptic ulcers, peripheral blood vessel disease, prostate cancer, esophageal reflux, renal insufficiency, rheumatoid arthritis, schizophrenia, sepsis, septic shock, serious systemic mycosis, small-cell lung cancer, spinal injuries, stomach cancer, systemic lupus erythematosus, transient ischemic attack, tuberculosis, valvular heart disease, vascular/multi-infarct dementia, trauma therapy, insomnia, arthritis, insufficiency of pituitary hormone secretion, pollakiuria, uremia, and neurodegenerative diseases.

In the screening, polypeptides of the invention can be used, or a receptor binding assay system using a constructed expression system of a recombinant polypeptide of the present invention can be used in order to screen compounds (such as peptides, proteins, non-peptide compounds, synthetic compounds, and fermented products) or their salts which modifiy the binding between polypeptides of the present invention and their receptors (compounds that promote or inhibit the activity of the polypeptides of the present invention). Such compounds include compounds with the receptor-mediated cell-stimulating activity (such as activity of promoting arachidonic acid release, acetylcholine release, intracellular $Ca^{2+}$ release, intracellular cAMP production, intracellular cGMP production, inositol phosphate production, changes in cell membrane potential, intracellular protein phosphorylation, c-fos activation, and decreases in pH) (that is, agonists of receptors of the polypeptides of the present invention), and compounds with no such cell-stimulating activity (that is, antagonists of receptors of the polypeptides of the present invention). To "modify the binding between polypeptides of the present invention and their receptors" means to either inhibit or promote the binding.

That is, the present invention provides:

a method for screening compounds, or their salts, that promote or inhibit the activity of polypeptides of the invention, characterized by the use of a polypeptide of the invention.

Specifically, the invention provides:

a method for screening compounds or their salts which modify the binding between polypeptides of the present invention and their receptors (compounds that promote or inhibit the activity of polypeptides in the invention), characterized by comparing (i) a case in which a polypeptide of the present invention is brought into contact with a receptor (or its salt) of the polypeptide of the invention, or a partial peptide of the receptor, and (ii) a case in which a polypeptide of the present invention and a test compound are brought into contact with a receptor (or its salt) of the polypeptide of the invention, or a partial peptide of the receptor.

The screening method of the present invention involves, for example, measurement and comparison of the binding amount of the polypeptide of the invention to a receptor (or its salt) of the polypeptide of the invention or a partial peptide of the receptor, or the cell-stimulating activity, etc. between (i) a case in which a polypeptide of the present invention is brought into contact with a receptor (or its salt) of the polypeptide of the invention, or a partial peptide of the receptor, and (ii) a case in which a polypeptide of the present invention and a test compound are brought into contact with a receptor (or its salt) of the polypeptide of the invention, or a partial peptide of the receptor.

Specific examples of such a screening method include:

(1) a method for screening a compound or its salt which modifies the binding between the polypeptide of the present invention and a receptor of the polypeptide of the invention (a compound that promotes or inhibits the activity of the polypeptide of the invention), characterized by comparative assay of the amount of the labeled polypeptide of the invention bound to the receptor (or its salt) of the polypeptide or the partial peptide (or its salt) of the receptor, between a case in which the labeled polypeptide of the invention is brought into contact with the receptor (or its salt) of the polypeptide of the invention, or the partial peptide (or its salt) of the receptor, and a case in which the labeled polypeptide of the invention and a test compound are brought into contact with the receptor (or its salt) of the polypeptide of the invention, or the partial peptide (or its salt) of the receptor;

(2) a method for screening a compound or its salt which modifies the binding between the polypeptide of the present invention and a receptor of the polypeptide of the invention (a compound that promotes or inhibits the activity of the polypeptide of the invention), characterized by comparative assay of the amount of the labeled polypeptide of the invention bound to a cell or membrane fraction thereof containing the receptor of the polypeptide of the invention, between a case in which the labeled polypeptide of the invention is brought into contact with the cell or membrane fraction thereof containing the receptor, and a case in which the labeled polypeptide of the invention and a test compound are brought into contact with the cell or membrane fraction thereof containing the receptor;

(3) a method for screening a compound or its salt which modifies the binding between the polypeptide of the present invention and a receptor of the polypeptide of the invention (a compound that promotes or inhibits the activity of the polypeptide of the invention), characterized by comparative assay of the amount of the labeled polypeptide of the invention bound to the receptor of the polypeptide of the invention, between a case in which the labeled polypeptide of the invention is brought into contact with the receptor expressed on cell membrane of a cultured transformant containing DNA coding for the receptor of the polypeptide of the invention, and a case in which the labeled polypeptide of the invention and a test compound are brought into contact with the receptor expressed on the cell membrane of the cultured transformant containing DNA coding for the receptor of the polypeptide of the invention;

(4) a method for screening a compound or its salt which modifies the binding between the polypeptide of the present invention and a receptor of the polypeptide of the invention (a compound that promotes or inhibits the activity of the polypeptide of the invention), characterized by comparative assay of the receptor-mediated cell-stimulating activity (such as activity of promoting or inhibiting arachidonic acid release, acetylcholine release, intracellular $Ca^{2+}$ release, intracellular cAMP production, intracellular cGMP production, inositol phosphate production, changes in cell membrane potential, intracellular protein phosphorylation, c-fos activation, and decreases in pH), between a case in which a compound that activates the receptor of the polypeptide of the invention (such as the polypeptide of the invention) is brought into contact with a cell containing the receptor of the polypeptide, and a case in which the compound that activates the receptor of the polypeptide and a test compound are brought into contact with a cell containing the receptor of the polypeptide; and

(5) a method for screening a compound or its salt which modifies the binding between the polypeptide of the present invention and a receptor of the polypeptide of the invention (a compound that promotes or inhibits the activity of the polypeptide of the invention), characterized by comparative assay of the receptor-mediated cell-stimulating activity (such as activity of promoting or inhibiting arachidonic acid release, acetylcholine release, intracellular $Ca^{2+}$ release, intracellular cAMP production, intracellular cGMP production, inositol phosphate production, changes in cell membrane potential, intracellular protein phosphorylation, c-fos activation, and decreases in pH), between a case in which a compound that activates the receptor of the polypeptide of the invention (such as the polypeptide of the invention) is brought into contact with the receptor expressed on cell membrane of a cultured transformant containing DNA coding for the receptor of the polypeptide of the invention, and a case in which the compound that activates the receptor of the polypeptide and a test compound are brought into contact with the receptor expressed on the cell membrane of the cultured transformant containing DNA coding for the receptor.

The screening method of the present invention is described specifically below.

The receptor of the polypeptide of the invention used in the screening method of the invention may be any that interact with the polypeptide of the invention as a ligand. Membrane fractions and the like of human or warm-blooded animal organs are preferred. However, because of the extreme difficulty in obtaining human organs in particular, the receptor of the polypeptide of the invention which is expressed in large amounts in a recombinant is suitable for use in the screening.

The aforementioned methods for producing the polypeptide of the present invention can be used to produce the receptor of the polypeptide of the invention.

When using a cell or membrane fraction thereof containing the receptor of the polypeptide of the invention in the screening method of the present invention, the cell and membrane fraction thereof can be prepared as described below.

When using a cell containing the receptor of the polypeptide of the invention, the cell may be fixed with glutaraldehyde, formalin, or the like. The cell can be fixed in accordance with a well-known method.

The cell containing the receptor of the polypeptide of the invention refers to a host cell in which such a receptor is expressed. Such a host cell includes *Escherichia coil, Bacillus subtilis*, yeasts, insect cells, and animal cells. The host cell expressing the receptor of the polypeptide of the invention can be obtained in the same manner as the methods for producing the transformant prepared with the expression vector for the polypeptide of the invention, as described above.

The cell membrane fraction refers to a fraction containing an abundance of cell membranes, which are obtained by a well-known method after the cells have been disrupted. Methods for disrupting cells include methods for crushing cells with a Potter-Elvehjem homogenizer, disruption with a Waring blender or a Polytron (manufactured by Kinematica), ultrasonic disruption, and disruption using a French press or the like, where the cells are discharged under pressure through narrow nozzles. Fractions of cell membranes are obtained primarily through fractionation with centrifugal force, such as fraction centrifugation or density gradient centrifugation. For example, cell lysates are centrifuged for a short period of time (usually about 1 to 10 minutes) at low speed (500 to 3,000 rpm), and the supernatant is then usually further centrifuged for 30 minutes to 2 hours at high speed (15,000 to 30,000 rpm), giving membrane fractions in the form of precipitate. Such membrane fractions contain an abundance of the expressed receptor of the polypeptide of the invention and membrane components such as cell phospholipids or membrane proteins.

The amount of the receptor of the polypeptide of the invention in the cell or membrane fraction having such a receptor should be $10^3$ to $10^8$ molecules, and more preferably $10^5$ to $10^7$ molecules per cell. The greater the amount expressed, the higher the polypeptide binding activity (specific activity) per membrane fraction, which not only allows the construction of a highly sensitive screening system but also allows the assay for large numbers of samples in the same lot.

A suitable receptor fraction and labeled polypeptide of the present invention can be used in the methods (1) through (3) above to screen for a compound that modifies the binding between the polypeptide of the invention and the receptor of the polypeptide (a compound that promotes or inhibits the activity of polypeptide of the invention). Preferred examples of the receptor fraction of the polypeptide of the present invention include native receptor fractions, and recombinant receptor fractions with equivalent activity. As used herein, "equivalent activity" means equivalent polypeptide binding activity and the like. Labeled polypeptides of the invention include labeled polypeptides and labeled analog compounds of the polypeptide. Examples include polypeptides of the present invention labeled with $^{3}H$, $^{125}I$, $^{14}C$, and $^{35}S$.

In order to screen a compound that modifies the binding between the polypeptide of the present invention and the receptor of the polypeptide of the invention, a receptor preparation can first be prepared by suspending cells or cell membrane fractions containing the receptor of the polypeptide of the invention in buffer suitable for screening. Examples of buffer include any that will not inhibit the binding between the polypeptide and the receptor, such as Tris-HCl buffer or phosphate buffer with a pH of 4 to 10 (and preferably a pH of 6 to 8). Surfactant such as CHAPS, Tween-80™ (by Kao-Atlas), digitonin, and deoxycholate can be added to the buffer to reduce non-specific binding. A protease inhibitor such as PMSF, leupeptin, E-64 (manufactured by Peptide Institute, Inc.), or pepstatin can also be added to inhibit the degradation of the polypeptides or receptors by proteases. A certain amount (5,000 cpm to 500,000 cpm) of labeled polypeptide of the present invention is added to 0.01 to 10 mL of the above receptor solution in the presence of $10^{-10}$ M to $10^{-7}$ M test compound. A reaction tube with an excess of unlabeled polypeptide of the invention is also prepared to determine the non-specific binding (NSB). The reaction is carried out for about 20 minutes to 24 hours, and preferably about 30 minutes to 3 hours, at a temperature of about 0°C to 50°C, and preferably about 4°C to 37°C. After the reaction, the reaction mixture is filtered through glass fiber filter paper or the like and is washed with a suitable amount of the same buffer, and the radioactivity remaining on the glass fiber filter paper is measured with a liquid scintillation counter or γ-counter. A test compound with a specific binding (B-NSB) of, for example, no more than 50%, can be selected as a candidate having the antagonist inhibitory activity, where 100% is the count ($B_0$-NSB) calculated by subtracting the non-specific binding (NSB) from the count in the absence of any competing substances ($B_0$).

In methods (4) and (5) above to screen for a compound that modifies the binding between the polypeptide of the present invention and the receptor of the polypeptide of the invention (a compound that promotes or inhibits the activity of polypeptides), cell-stimulating activity mediated by the receptor of the polypeptide (such as activity of promoting arachidonic acid release, acetylcholine release, intracellular $Ca^{2+}$ release, intracellular cAMP production, intracellular cGMP production, inositol phosphate production, changes in cell membrane potential, intracellular protein phosphorylation, c-fos activation, and decreases in pH) can be assayed using a common method or a commercially available kit. Specifically, the cell containing the receptor of the polypeptide is first cultured in multi-well plates or the like. In performing the screening, the medium is replaced with fresh medium or a suitable buffer that is not toxic to the cells, a test compound or the like is added, the mixture is incubated for a certain period of time, the cell is then extracted or the supernatant is collected, and the product is quantified according to a variety of methods. When the production of a substance indicating the cell-stimulating activity (such as arachidonic acid) is difficult to detect because of degrading enzymes in the cells, the assay may be performed in the presence of an inhibitor for such enzymes. For example, activity of inhibiting cAMP production can be detected through inhibitory effect on the cell in which basic cAMP production is increased by forskolin or the like.

Suitable Cells expressing the receptor of the polypeptide of the invention are necessary for screening by assay of the cell-stimulating activity. Preferred cells expressing the receptor of the polypeptide include clonal cell lines expressing such a receptor, as described above.

Examples of a test compound include peptides, proteins, non-peptide compounds, synthetic compounds, fermented products, cell extracts, plant extracts, and animal tissue extracts. Such a compound may be a novel compound or a well-known compound.

A screening kit for a compound or its salt that modifies the binding between the polypeptide of the present invention or its precursor protein and a receptor of the polypeptide of the present invention (a compound that promotes or inhibits the activity of the polypeptide of the invention) comprises a receptor(or its salt)of the polypeptide of the present invention, a partial peptide (or its salt) of the receptor of the polypeptide of the present invention, a cell containing the receptor of the polypeptide of the present invention, a cell membrane fraction containing the receptor of the polypeptide of the present invention, and the polypeptide of the present invention.

The following are examples of such a screening kit.

1. Screening reagents:

(1) Assay buffer and washing buffer

Hanks' balanced salt solution(Gibco) supplemented with 0.05% bovine serum albumin (by Sigma).

This can be sterilized by filtration with a filter having a pore size of 0.45 μm, and stored at 4°C, or it can be prepared at the time of use.

(2) a receptor preparation of the polypeptide of the invention

CHO cells expressing a receptor of the polypeptide of the present invention, which are plated at $5 \times 10^5$ cells/well in 12-well plates, and cultured for 2 days at 37°C in 5% $CO_2$ and 95% air

(3) Labeled polypeptide of the invention

The polypeptide of the present invention labeled with {$^3$H}, {$^{125}$I}, {$^{14}$C}, {$^{35}$S} or the like

Stored the labeled polypeptide dissolved in a suitable solvent or buffer at 4°C or -20°C, and diluted with assay buffer to 1 μM at the time of use

(4) Standard polypeptide solution

The polypeptide of the present invention is dissolved to a concentration of 1 mM in PBS containing 0.1% bovine serum albumin(Sigma), and stored at -20°C.

2. Assay:

(1) Cells expressing a receptor of the polypeptide of the present invention, which have been cultured in 12-well tissue culture plates, are washed twice with 1 mL assay buffer, and 490 μL assay buffer is then added per well.
(2) 5 μL of $10^{-3}$ to $10^{-10}$ M test compound solution is added, 5 μL labeled polypeptide of the invention is then added, and a reaction is made for 1 hour at room temperature. 5 μL of $10^{-3}$ M polypeptide of the invention is added instead of the test compound to determine the non-specific binding.
(3) The reaction solution is removed, and the cells are washed 3 times with 1 mL washing buffer. The labeled polypeptide of the invention bound to the cells is dissolved in 0.2 N NaOH-1% SDS and mixed with 4 mL liquid Scintillator A (Wako Pure Chemicals).
(4) The radioactivity is assayed using a liquid scintillation counter (Beckman), and the percent maximum binding (PMB) is determined using the following equation 1.

[Equation 1]

$$PMB = \{(B\text{-}NSB)/(B_0\text{-}NSB)\} \times 100$$

PMB: percent maximum binding
B: value when sample added
NSB: non-specific binding
$B_0$: maximum binding

A compound or its salt obtained using the screening method or the screening kit of the invention means a compound that modifies (promotes or inhibits) the binding between the polypeptide of the invention and a receptor of the polypeptide of the invention (a compound that promotes or inhibits the activity of polypeptide of the invention), specifically, a compound or its salt having the cell-stimulating activity mediated by the receptor (referred to as an agonist of the receptor of the polypeptide of the invention), or a compound having no such cell-stimulating activity (referred to as an antagonist of the receptor of the polypeptide of the invention). Examples of such a compound include peptides, proteins, non-peptide compounds, synthetic compounds, and fermented products. Such a compound may be a novel compound or a well-known compound.
The following specific methods (i) or (ii) should be followed to evaluate whether the compound is an agonist or antagonist of the receptor of the polypeptide of the invention.

(i) Binding assay is performed as indicated in the screening method of (1) through (3) above to obtain a compound that modifies (inhibits, in particular) the binding between the polypeptides of the invention and the receptor of the polypeptide of the invention, and it is then determined whether or not the compound has the cell-stimulating activity mediated by the aforementioned receptor. A compound or its salt having the cell-stimulating activity is an agonist of the receptor of the polypeptide of the invention, while a compound or its salt having no such activity is an antagonist of the receptor.
(ii)

(a) Test compounds are brought into contact with cells containing the receptor of the polypeptide of the invention to assay the cell-stimulating activity mediated by the aforementioned receptor. A compound or its salt having the cell-stimulating activity is a agonist of the receptor of the polypeptide of the invention.
(b) The cell-stimulating activity mediated by the receptor of the polypeptide of the invention are comparatively assayed between a case in which a compound that activates the receptor of the polypeptide (such as the polypeptide of the invention or an agonist of the receptor of the polypeptide) are brought into contact with cells containing the receptor of the polypeptide of the invention, and a case in which a test compound and a compound that activates the receptor of the polypeptide (such as the polypeptide of the invention or an agonist of the receptor of the polypeptide) are brought into contact with cells containing the receptor of the polypeptide of the invention. A compound or its salt that is capable of reducing the cell-stimulating activity caused by the compound that activates the receptor of the polypeptide is an antagonist of the receptor of the polypeptide of the invention.

The agonist of the receptor of the polypeptide of the invention has activity similar to the physiological activity of the polypeptide of the invention on the receptor of the polypeptide, and is therefore useful as a safe drug with low toxicity, in the same manner as the polypeptide of the invention.

Conversely, the antagonist of the receptor of the polypeptide of the invention is capable of inhibiting the physiological activity of the polypeptide of the invention on the receptor of the polypeptide, and is therefore useful as a safe drug with low toxicity for inhibiting such receptor activity.

A compound or its salt obtained using the screening method or screening kit of the invention can be selected from peptides, proteins, non-peptide compounds, synthetic compounds, fermented products, cell extracts, plant extracts, animal tissue extracts, plasma, or the like, and is a compound that promotes or inhibits functions of the polypeptide of the invention.

Examples of salts of such a compound are the same as the examples of salts given for the polypeptide of the present invention above.

Common procedures can be followed when a compound obtained using the screening method or kit of the invention is used as the remedy and prophylactic described above. For example, it can be used in the form of tablet, capsule, elixir, microcapsule, sterile solution, suspension, or the like in the same manner as drugs containing the polypeptide of the invention, as described above.

Because such a preparation is safe and has low toxicity, it can be administered, for example, to humans and warm-blooded animals (such as mice, rats, rabbits, sheep, pigs, cows, horses, birds, cats, dogs, monkeys, and chimpanzees).

The dosage of such a compound and its salt will vary depending on the activity, the target disease, purpose of administration, route of administration or the like, but the daily adult oral dosage of the compound that promotes the function of the polypeptide of the invention for the treatment of neurological diseases, for example, may generally range from about 0.1 to 100 mg, preferably from about 1.0 to 50 mg, and even more preferably from about 1.0 to 20 mg (per 60 kg body weight). The single parenteral dose of such a compound will vary depending on the purpose of administration, target disease, and the like, but in the form of an injection for adults, for example, the daily dosage of the compound that promotes the function of the polypeptide of the invention for treatment of neurological diseases may usually range from about 0.01 to about 30 mg, preferably about 0.1 to about 20 mg, and even more preferably about 0.1 to about 10 mg at a time (per 60 kg body weight), given by intravenous injection. Doses for animals can be given as calculated per 60 kg body weight.

[3] Quantification of the Polypeptides of the Invention

Antibodies against polypeptides of the invention (sometimes referred to below simply as antibodies of the invention) specifically recognize polypeptides of the invention, and can therefore be used to quantify polypeptides of the invention in analyte, particularly assay by sandwich immunoassay.

Specifically, the invention is intended to provide:

(i) a method for quantifying polypeptides of the invention in analyte, characterized by bringing about a competitive reaction of antibody of the invention with analyte and labeled polypeptides of the invention to determine the proportion of labeled polypeptides of the invention binding to the antibody; and

(ii) a method for quantifying polypeptides of the invention in analyte, characterized by bringing about simultaneous or continuous reaction of analyte with antibody of the present invention insolubilized on a carrier and other labeled antibody of the invention, and then assaying the activity of the labeled antibody on the insolubilization carrier.

In the method of quantification in (ii), one antibody should recognize the N terminal of polypeptides of the invention, and the other antibody should react with the C terminal of polypeptides of the invention.

Polypeptides of the invention can be quantified using monoclonal antibodies against polypeptides of the invention, but they can also be detected by tissue staining, or the like. For that purpose, the antibody molecules themselves may be used, and $F(ab')_2$, Fab', or Fab fractions of antibody molecules may also be used.

Quantification of polypeptides of the present invention using antibodies of the invention is not particularly limited, and any method of quantification can be used in which the amount of antibody, antigen, or antibody-antigen complex relative to the amount of antigen (such as the amount of polypeptide) in the analyte is detected by chemical or physical means, and is calculated from a standard curve prepared using a standard containing a known amount of antigen. For example, nephrometry, competitive methods, immunometric methods, and sandwich methods are suitable for use, although the use of a sandwich method, as described below, is preferred in terms of sensitivity and specificity.

Examples of labels for use in assays using labeled substances include radioisotopes, enzymes, fluorescent substances, and luminescent substances. Examples of radioisotopes include [$^{125}I$], [$^{131}I$], [$^{3}H$] and [$^{14}C$]. Examples of enzymes include those that are stable and that have high specific activity, such as (β-galactosidase, β-glucosidase, alkali phosphatase, peroxidase, and malate dehydrogenase. Examples of fluorescent substances include fluorescamine and fluorescein isothiocyanate. Examples of luminescent substances include luminol, luminol derivatives, luciferin, and lucigenin. A biotin-avidin system may also be used for binding between antibody or antigen and a label.

Physical adsorption may be employed for the insolubilization of antigens or antibodies, and methods employing chemical bonding may also normally be used for the insolubilization or immobilization of polypeptides, enzymes, or the like. Examples of carriers include insoluble polysaccharides such as agarose, dextran and cellulose, synthetic resins such as polystyrene, polyacrylamide and silicone, and glass or the like.

In a sandwich method, the test liquid is allowed to react with insolubilized monoclonal antibody of the present invention (first reaction), separate labeled monoclonal antibody of the present invention is allowed to react (the second reaction), and the activity of the label on the insoluble carrier is then assayed so as to quantify the amount of polypeptides of the present invention in the analyte. The first and second reactions may be carried out in reverse order, simultaneously, or while staggered. The label and method of insolubilization can be based on those described above. In sandwich immunoassay, the antibody used for the labeled antibody or solid phase antibody need not necessarily be one type; a mixture of two or more types of antibody may be used to improve assay sensitivity or the like.

The monoclonal antibodies of the present invention which are used in the first and second reactions in the sandwich assay of polypeptides of the invention should have different binding sites for polypeptides of the invention. Specifically, the antibodies used in the first and second reactions should recognize a region other than C terminal region, such as the N terminal region, for example, whenever the antibody used in the second reaction recognizes the C terminal region of polypeptides in the invention.

Monoclonal antibodies of the present invention may be used in assay systems other than sandwich assay, such as competitive methods, immunometric methods, and nephrometry.

In competitive methods, labeled antigen and antigen in an analyte are allowed to undergo competitive reaction with antibody, the unreacted labeled antigen (F) and the labeled antigen (B) binding to the antibody are then separated (B/F separation), and the amount of label in either B or F is determined so as to quantify the amount of antigen in the analyte. This method of reaction can entail the use of a liquid phase method in which insoluble antibody is used as the antibody, and polyethylene glycol and secondary antibody against the aforementioned antibody is used in the B/F separation, and a solid phase in which immobilized antibody is used as primary antibody, or the primary antibody is soluble and immobilized antibody is used as the secondary antibody.

In immunometric methods, immobilized antigen and antigen in analyte are allowed to undergo competitive reaction with a given amount of labeled antibody, and the solid and liquid phases are then separated, or the antigen in the analyte is allowed to react with an excess of labeled antibody, the immobilized antigen is then added to allow the unreacted labeled antibody bind to the solid phase, and the solid and liquid phases are then separated. The amount of label in either phase is then determined to quantify the amount of antigen in the analyte.

In nephrometry, the amount of insoluble precipitate produced as a result of an antigen-antibody reaction in gel or solution is measured. Laser nephrometry based on laser scattering or the like is suitable for use in cases involving trace amounts of antigen in analyte which result in only minute amounts of precipitate.

No special conditions, operations or the like need to be established in order to apply these individual immunoassay methods to the quantification method of the present invention. The polypeptide assay system of the present invention should be constructed based on common technical considerations known to those having ordinary skill in the art for the usual conditions and operations in the individual methods above. The general technical details can be found in references, documents, and the like.

Examples include Hiroshi Irie, Ed., *Radioimmunoassay* (published by Kodansha (1974)); Hiroshi Irie, Ed., *Radioimmunoassay, Part II* (published by Kodansha (1979)); Eiji Ishikawa et al. , Ed., *Enzyme Immunoassay* (published by Igaku Shoin (1978)); Eiji Ishikawa et al., Ed., *Enzyme Immunoassay* (Second Edition) (published by Igaku Shoin (1982)); Eiji Ishikawa et al., Ed., *Enzyme Immunoassay* (Third Edition) (published by Igaku Shoin (1987)); *Methods in Enzymology* Vol. 70 (Immunochemical Techniques (Part A)); ibid. Vol. 73 (Immunochemical Techniques (Part B)); ibid. Vol. 74 (Immunochemical Techniques (Part C)); ibid. Vol. 84 (Immunochemical Techniques (Part D: Selected Immunoassays)); ibid. Vol. 92 (Immunochemical Techniques (Part E: Monoclonal Antibodies and General Immunoassay Methods)); ibid. Vol. 121 (Immunochemical Techniques (Part I: Hybridoma Technology and Monoclonal Antibodies)) (Academic Press).

Antibodies of the present invention can be used in the manner described above for the sensitive quantification of polypeptides of the present invention.

Decreases or increases in the concentration of polypeptides of the invention can be detected through the quantification of the concentration of polypeptides of the present invention using antibodies of the present invention in order to permit the diagnosis of the presence or the high possibility of future onset of diseases such as hypertension, autoimmune diseases, cardiac failure, cataracts, glaucoma, acute bacterial meningitis, acute myocardial infarction, acute pancreatitis, acute viral encephalitis, adult respiratory distress syndrome, alcoholic hepatitis, Alzheimer's disease, asthma, atherosclerosis, atopic dermatitis, bacterial pneumonia, bladder carcinoma, bone fractures, breast cancer, hyperexia, adphagia, burn treatment, uterine cancer, chronic lymphatic leukemia, chronic myeloid leukemia, chronic pancreatitis, hepatic cirrhosis, colon cancer (colorectal cancer), Crohn's disease, dementia, diabetic complications, diabetic nephropathy, diabetic neuropathy, diabetic retinopathy, gastritis, *Helicobacter pylori* infection, hepatic insufficiency, hepatitis A, hepatitis B, hepatitis C, hepatitis, Herpes simplex, Herpes zoster, Hodgkin's disease, AIDS, human papilloma viral infections, hypercalcemia, hypercholesterolemia, hypertriglyceridemia, hyperlipemia, infections, influenza viral infections, insulin-dependent diabetes (type I), invasive Staphylococcus infection, malignant melanoma, metastatic cancer, multiple myeloma, allergic rhinitis, nephritis, non-Hodgkin's lymphoma, non-insulin-dependent diabetes (type II), non-small cell lung cancer, organ transplants, osteoarthropy, osteomalacia, osteopenia, osteoporosis, ovarian cancer, bone Paget disease, peptic ulcers, peripheral blood vessel disease, prostate cancer, esophageal reflux, renal insufficiency, rheumatoid arthritis, schizophrenia, sepsis, septic shock, serious systemic mycosis, small-cell lung cancer, spinal injuries, stomach cancer, systemic lupus erythematosus, transient ischemic attack, tuberculosis, valvular heart disease, vascular/multi-infarct dementia, trauma therapy, insomnia, arthritis, insufficiency of pituitary hormone secretion, pollakiuria, uremia, and neurodegenerative diseases.

The antibodies of the invention can also be used to detect polypeptides of the invention present in analytes such as bodily fluids and tissue. They can also be used to prepare antibody columns for use in the purification of polypeptides of the invention, to detect polypeptides of the invention in fractions during purification, to analyze the behavior of polypeptides of the invention in analyte cells, and so forth.

[4] Genetic Diagnostic Agents

DNA of the present invention can be used as probe, for example, to detect abnormalities (genetic abnormalities) in DNA or mRNA coding for polypeptides of the invention in humans or warm-blooded animals (such as rats, mice, guinea pigs, rabbits, birds, sheep, pigs, cows, horses, cats, dogs, and monkeys), and can thus be useful as genetic diagnostic agents for ascertaining damage to, variation in, or reduced expression of DNA or mRNA, or under- or over-expression of DNA or mRNA.

Such genetic diagnostics using DNA of the invention can be carried out by a well-known method, such as Northern hybridization or PCR-SSCP (*Genomics,* **5**, 874-879 (1989); and *Proceedings of the National Academy of Sciences of the United States of America,* **86**, 2766-2770 (1989)).

Under- or over-expression revealed by Northern hybridization can permit the diagnosis of the presence or the possibility of future onset of diseases such as hypertension, autoimmune diseases, cardiac failure, cataracts, glaucoma, acute bacterial meningitis, acute myocardial infarction, acute pancreatitis, acute viral encephalitis, adult respiratory distress syndrome, alcoholic hepatitis, Alzheimer's disease, asthma, atherosclerosis, atopic dermatitis, bacterial pneumonia, bladder carcinoma, bone fractures, breast cancer, hyperexia, adphagia, burn treatment, uterine cancer, chronic lymphatic leukemia, chronic myeloid leukemia, chronic pancreatitis, hepatic cirrhosis, colon cancer (colorectal cancer), Crohn's disease, dementia, diabetic complications, diabetic nephropathy, diabetic neuropathy, diabetic retinopathy, gastritis, *Helicobacter pylori* infection, hepatic insufficiency, hepatitis A, hepatitis B, hepatitis C, hepatitis, Herpes simplex, Herpes zoster, Hodgkin's disease, AIDS, human papilloma viral infections, hypercalcemia, hypercholesterolemia, hypertriglyceridemia, hyperlipemia, infections, influenza viral infections, insulin-dependent diabetes (type I), invasive Staphylococcus infection, malignant melanoma, metastatic cancer, multiple myeloma, allergic rhinitis, nephritis, non-Hodgkin's lymphoma, non-insulin-dependent diabetes (type II), non-small cell lung cancer, organ transplants, osteoarthropy, osteomalacia, osteopenia, osteoporosis, ovarian cancer, bone Paget disease, peptic ulcers, peripheral blood vessel disease, prostate cancer, esophageal reflux, renal insufficiency, rheumatoid arthritis, schizophrenia, sepsis, septic shock, serious systemic mycosis, small-cell lung cancer, spinal injuries, stomach cancer, systemic lupus erythematosus, transient ischemic attack, tuberculosis, valvular heart disease, vascular/multi-infarct dementia, trauma therapy, insomnia, arthritis, insufficiency of pituitary hormone secretion, pollakiuria, uremia, and neurodegenerative diseases.

[5] Therapeutic Drugs Containing Antisense DNA

Antisense DNA capable of complementarily binding to DNA of the present invention to inhibit the expression of such DNA can be used as an agent in the treatment and prevention of diseases such as hypertension, autoimmune diseases, cardiac failure, cataracts, glaucoma, acute bacterial meningitis, acute myocardial infarction, acute pancreatitis, acute viral encephalitis, adult respiratory distress syndrome, alcoholic hepatitis, Alzheimer's disease, asthma, atherosclerosis, atopic dermatitis, bacterial pneumonia, bladder carcinoma, bone fractures, breast cancer, hyperexia, adphagia, burn treatment, uterine cancer, chronic lymphatic leukemia, chronic myeloid leukemia, chronic pancreatitis, hepatic cirrhosis, colon cancer (colorectal cancer), Crohn's disease, dementia, diabetic complications, diabetic nephropathy, diabetic neuropathy, diabetic retinopathy, gastritis, *Helicobacter pylori* infection, hepatic insufficiency, hepatitis A, hepatitis B, hepatitis C, hepatitis, Herpes simplex, Herpes zoster, Hodgkin's disease, AIDS, human papilloma viral infections, hypercalcemia, hypercholesterolemia, hypertriglyceridemia, hyperlipemia, infections, influenza viral infections, insulin-dependent diabetes (type I), invasive Staphylococcus infection, malignant melanoma, metastatic cancer, multiple myeloma, allergic rhinitis, nephritis, non-Hodgkin's lymphoma, non-insulin-dependent diabetes (type II), non-small cell lung cancer, organ transplants, osteoarthropy, osteomalacia, osteopenia, osteoporosis, ovarian cancer, bone Paget disease, peptic ulcers, peripheral blood vessel disease, prostate cancer, esophageal reflux, renal insufficiency, rheumatoid arthritis, schizophrenia, sepsis, septic shock, serious systemic mycosis, small-cell lung cancer, spinal injuries, stomach cancer, systemic lupus erythematosus, transient ischemic attack, tuberculosis, valvular heart disease, vascular/multi-infarct dementia, trauma therapy, insomnia, arthritis, insufficiency of pituitary hormone secretion, pollakiuria, uremia, and neurodegenerative diseases.

In such antisense DNA applications, the antisense DNA can be used according to common methods, either by itself or after being incorporated into suitable vectors such as retrovirus vectors, adenovirus vectors, and adenovirus-associated virus vectors. Such antisense DNA can be given, either as such or in the form of a preparation combined with a physiologically acceptable carrier such as an adjuvant to facilitate ingestion, by means of a gene gun or a catheter such as a hydrogel catheter.

Antisense DNA can also be used as a diagnostic oligonucleotide probe to check for the presence or the expression of DNA of the present invention in tissue or cells.

[6] Therapeutic Drugs Containing Antibodies of the Invention

Antibodies of the invention which have activity of neutralizing polypeptides of the invention can be used as drugs for the treatment and prevention of diseases such as hypertension, autoimmune diseases, cardiac failure, cataracts, glaucoma, acute bacterial meningitis, acute myocardial infarction, acute pancreatitis, acute viral encephalitis, adult respiratory distress syndrome, alcoholic hepatitis, Alzheimer's disease, asthma, atherosclerosis, atopic dermatitis, bacterial pneumonia, bladder carcinoma, bone fractures, breast cancer, hyperexia, adphagia, burn treatment, uterine cancer, chronic lymphatic leukemia, chronic myeloid leukemia, chronic pancreatitis, hepatic cirrhosis, colon cancer (colorectal cancer), Crohn's disease, dementia, diabetic complications, diabetic nephropathy, diabetic neuropathy, diabetic retinopathy, gastritis, *Helicobacter pylori* infection, hepatic insufficiency, hepatitis A, hepatitis B, hepatitis C, hepatitis, Herpes simplex, Herpes zoster, Hodgkin's disease, AIDS, human papilloma viral infections, hypercalcemia, hypercholesterolemia, hypertriglyceridemia, hyperlipemia, infections, influenza viral infections, insulin-dependent diabetes (type I), invasive Staphylococcus infection, malignant melanoma, metastatic cancer, multiple myeloma, allergic rhinitis, nephritis, non-Hodgkin's lymphoma, non-insulin-dependent diabetes (type II), non-small cell lung cancer, organ transplants, osteoarthropy, osteomalacia, osteopenia, osteoporosis, ovarian cancer, bone Paget disease, peptic ulcers, peripheral blood vessel disease, prostate cancer, esophageal reflux, renal insufficiency, rheumatoid arthritis, schizophrenia, sepsis, septic shock, serious systemic mycosis, small-cell lung cancer, spinal injuries, stomach cancer, systemic lupus erythematosus, transient ischemic attack, tuberculosis, valvular heart disease, vascular/multi-infarct dementia, trauma therapy, insomnia, arthritis, insufficiency of pituitary hormone secretion, pollakiuria, uremia, and neurodegenerative diseases.

Such agents containing antibodies of the present invention for the treatment and prevention of the aforementioned drugs can be given orally or parenterally in unmodified liquid form or in the form of suitable medicinal compositions to humans or warm-blooded animals (such as rats, rabbits, sheep, pigs, cows, cats, dogs, and monkeys). The dosage of will vary depending on the purpose of administration, target disease, symptoms, route of administration or the like, but the single adult dosage of antibodies of the present invention for the treatment of neurological diseases, for example, may generally range from about 0.01 to 20 mg/kg, preferably from about 0.1 to 10 mg/kg, and even more preferably from about 0.1 to 5 mg, to be given by intravenous injection about 1 to 5 times a day, and preferably about 1 to 3 times a day. A dosage based on this can be given for other types of parenteral administration or oral administration. The dosage may be increased if symptoms are particularly serious.

Antibodies of the invention can be given as such or in the form of suitable medicinal compositions. Medicinal compositions used for the administration described above include the above or their salts with pharmaceutically acceptable carriers, diluents, or excipients. Such compositions may be provided in the form of preparations suitable for oral or parenteral administration.

That is, they can be used in the form of compositions for oral administration, specifically, tablets (including sugar-coated tablets and film-coated tablets), pills, granules, dispersions, capsules (including soft capsules), syrups, emulsions, and suspensions. Such compositions can be produced by a well-known method, and can include carriers, diluents, or excipients commonly used in the pharmaceutical field. Examples of carriers and excipients for tablets include lactose, starch, sucrose, and magnesium stearate.

Examples of compositions for parenteral use include injections, and suppositories. Injections include intravenous injections, subcutaneous injections, intracutaneous injections, intramuscular injections, and drip infusions. Such injections can be produced in accordance with a well-known method, such as the aforementioned antibodies or their salts dissolved, suspended, or emulsified in sterile aqueous or oleaginous liquids commonly used in injections. Aqueous liquids for injection include physiological saline and isotonic solutions containing glucose or other adjuvants, and may be used in combination with appropriate dissolution aids such as alcohols (such as ethanol), polyalcohols (such as propylene glycol and polyethylene glycol), and nonionic surfactants (such as Polysorbate 80™ and HCO-50 (polyoxyethylene (50 mol) adduct of hydrogenated castor oil)) may also be used. Oleaginous liquids include sesame oil and soybean oil, and may be used in combination with dissolution aids such as benzyl benzoate and benzyl alcohol. Suitable ampules are usually aseptically filled with the resulting injection liquid. Suppositories for rectal administration may be prepared by mixing the aforementioned antibodies or salts with a common suppository base.

The aforementioned oral and parenteral medicinal compositions may be prepared in the unit dose forms suitable for the dosage of active ingredient. Such unit dose forms include tablets, pills, capsules, injections (ampules), and suppositories, and should usually contain antibody in an amount of 5 to 500 mg per unit dose form, and more preferably 5 to 100 mg in injections and 10 to 250 mg in other formulations.

The aforementioned compositions may contain other active ingredients, provided that their combination with the aforementioned antibodies does not result in any unfavorable interaction.

[7] Transgenic Animals

The invention is also intended to provide non-human mammals with exogenous DNA coding for polypeptides of the present invention (sometimes referred to below simply as exogenous DNA) or mutant DNA thereof (sometimes referred to below simply as exogenous mutant DNA).

Specifically, the present invention is intended to provide:

(1) non-human mammals with exogenous DNA of the present invention or mutant DNA thereof;
(2) animals as described in (1) above, wherein the non-human mammal is a rodent;
(3) animals as described in (2) above, wherein the rodent is a mouse; and
(4) recombinant vectors which contain exogenous DNA or mutant DNA of the present invention, and which are capable of expression in mammals.

Non-human mammals with exogenous DNA or mutant DNA thereof in the invention (referred to below as transgenic animals of the invention) can be prepared by transferring the target DNA by means of calcium phosphate, electroporation, lipofection, agglutination, microinjection, particle gun, or DEAE-dextran to germinal cells or the like, including fertilized or unfertilized eggs, or spermatozoa or their primordial cells, preferably at the embryonic stage (and more preferably at the single cell or fertilized egg cell stage, or generally within the 8-cell stage). Such methods for transferring DNA can be employed to transfer target exogenous DNA of the invention to somatic cells, the organs of organisms, tissue cells, or the like for use in cell culture, tissue culture, or the like, and the cells can be fused by common methods of cell fusion with the aforementioned germinal cells to create transgenic animals of the invention.

Examples of non-human mammals include cows, pigs, sheep, goats, rabbits, dogs, cats, guinea pigs, hamsters, mice, and rats. Animals that are preferred among these for the purposes of preparing disease animal models include rodents, which are characterized by relatively rapid ontogeny and life cycle, and are easy to breed, particularly mice (such as pure strains like C57BL/6 and DBA2, and hybrid strains like B6C3$F_1$, $BDF_1$, B6D2$F_1$, BALB/c, and ICR) or rats (such as Wistar and SD).

"Mammals," as used in the context of recombinant vectors capable of expression in mammals, include the non-human mammals noted above, as well as humans.

The exogenous DNA of the present invention refers not to DNA of the present invention which is inherent to non-human mammals, but refers to DNA of the present invention which has been isolated and extracted from mammals.

Examples of mutant variant DNA of the present invention include that produced by variations (such as mutations) in the base sequence of the original DNA of the present invention, specifically, DNA with added or deleted bases, substitutions with other bases, or the like, as well as abnormal DNA.

Abnormal DNA means DNA causing the expression of abnormal polypeptides in the present invention, such as DNA causing the expression of polypeptides inhibiting the function of normal polypeptides of the invention.

Exogenous DNA of the invention may be from a mammal of either the same or different species of the target animal. To transfer DNA of the invention to a target animal, it is generally beneficial to use DNA in the form of a DNA construct which is ligated downstream of a promoter and is capable of expressing the DNA in animal cells. For example, when transferring human DNA of the present invention, DNA constructs (such as vectors) which comprise human DNA of the present invention ligated downstream of various promoters and which are capable of expressing the DNA of various mammals (such as rabbits, dogs, cats, guinea pigs, hamsters, rats, and mice) having DNA of the present invention with high homology therewith can be microinjected to fertilized eggs of the target mammals, such as mouse fertilized eggs, so as to construct a transgenic mammal with high expression of DNA of the present invention.

Examples of expression vectors for polypeptides of the invention include *E. coli* plasmids, *B. subtilis* plasmids, yeast plasmids, λ-phages and other bacteriophages, retroviruses such as Moloney leukemia virus, and animal viruses such as vaccinia virus and baculovirus. Preferred plasmids include *E. coli* plasmids, *B. subtilis* plasmids, and yeast plasmids.

Examples of promoters that regulate the expression of such DNA include (1) virus (such as simian virus, cytomegalovirus, Moloney leukemia virus, JC virus, papilloma virus, and poliovirus) DNA promoters; (2) mammal (such as human, rabbit, dog, cat, guinea pig, hamster, rat, and mouse) promoters, such as albumin, insulin II, uroplakin II, elastase, erythropoietin, endothelin, muscle creatine kinase, glial fibrillary acidic protein, glutathione S-transferase, platelet-derived growth factor β, keratin K1, K10, and K14, collagen type I and type II, cyclic AMP-dependent protein kinase βI subunit, dystrophin, tartaric acid-resistant alkaline phosphatase, atrial natriuretic factor, endothelial receptor tyrosine kinase (commonly abbreviated as Tie2), sodium/potassium-exchanging adenosine triphosphatase (Na, K-ATPase), neurofilament light chain, metallothionein I and IIA, metalloprotease I tissue inhibitor, MHC Class I antigen (H-2L), H-ras, renin, dopamine β-hydroxylase, thyroid peroxidase (TPO), polypeptide chain elongation factor 1α (EF-1α), β actin, α and β-myosin heavy chain, myosin light chains 1 and 2, myelin basic protein, thyroglobulin, Thy-1, immunoglobulin, H chain variable region (VNP), serum amyloid P component, myoglobin, troponin C, smooth muscle α-actin, preproenkephalin A, and vasopressin. Preferable promoters are promoters conducive to high expression throughout the entire body, such as cytomegalovirus promoter, human polypeptide chain elongation factor 1α (EF-1α) promoter, and human and chicken β-actin promoters.

The aforementioned vectors should have a sequence for terminating the transcription of the target mRNA (generally called the terminator) in the transgenic mammal, examples of which include DNA sequences of viruses and various mammals, preferably simian virus SV40 terminator or the like.

A splicing signal, enhancer region, a portion of eukaryotic DNA intron, or the like can be ligated upstream of the 5'-end of the promoter region, between the promoter region and the translated region, or downstream of the 3'-end of the translated region, depending on the purpose, in order to ensure higher expression of the target exogenous DNA.

The translated region of normal polypeptides of the present invention can be obtained in the form of either all or part of genomic DNA from a variety of commercially available genomic DNA libraries and DNA from humans or various mammals (such as rabbits, dogs, cats, guinea pigs, hamsters, rats, and mice), or by using as starting material complementary DNA prepared by a common method from RNA of liver, kidneys, thyroid cells or fibroblasts. Exogenous abnormal DNA can be obtained by preparing translated regions in which point mutations have been induced in the translated region of normal polypeptides obtained from the aforementioned cells or tissue.

The translated region can be prepared by common DNA engineering methods in which a DNA construct capable of expression in transgenic animals is ligated downstream of the a promoter such as the above and upstream of the transcription termination site as desired.

The transfer of exogenous DNA of the present invention at the fertilized egg cell stage can be carried out in such a way as to ensure its presence in all germinal cells and somatic cells of the target mammal. The presence of exogenous DNA of the invention in the germinal cells of the transgenic animal means that all subsequent generations of the transgenic animal will have the exogenous DNA of the invention in all their germinal cells and somatic cells. The offspring of animals of this line inheriting the exogenous DNA of the invention will have the exogenous DNA of the present invention in all their germinal cells and somatic cells.

Non-human mammals with the normal exogenous DNA of the present invention can be mated to verify stable retention of the exogenous DNA, and can be bred and raised in the usual breeding environment as animals conserving the DNA.

The transfer of exogenous DNA of the invention at the fertilized oocyte stage can be carried out in such a way as to ensure its excess presence in all germinal cells and somatic cells of the target mammal. The excess presence of exogenous DNA of the invention in the germinal cells of the transgenic animal means that all subsequent generations of the transgenic animal will have an excess of the exogenous DNA of the invention in all their germinal cells and somatic cells. The offspring of animals of this line inheriting the exogenous DNA of the invention will have an excess of the exogenous DNA of the present invention in all their germinal cells and somatic cells.

Homozygous animals of both sexes having the DNA in both homologous chromosomes can be mated and bred in such a way as to ensure all offspring have an excess of such DNA.

Non-human mammals with normal DNA of the present invention are characterized by high expression of the DNA, and may ultimately develop disorders involving hyperfunction of polypeptide of the present invention as a result of the promotion of the function of normal endogenous DNA, making them useful as disease model animals. For example, normal transgenic animals of the present invention can be used to elucidate the mechanisms of disorders associated with polypeptides of the invention or hyperfunction of polypeptides of the invention, and to study methods for treating such diseases.

Mammals with normal exogenous DNA of the invention may also have exacerbated symptoms associated with free polypeptides of the invention, and can thus be used to screen drugs for the treatment of diseases associated with polypeptides of the invention.

Non-human mammals with abnormal exogenous DNA of the present invention, meanwhile, can be mated to verify stable retention of the exogenous DNA, and can be bred and raised in the usual breeding environment as animals conserving the DNA. The target exogenous DNA can also be incorporated in the aforementioned plasmids for use as starting material. DNA constructs with promoters can be prepared by common DNA engineering techniques. The transfer of abnormal DNA of the invention at the fertilized oocyte stage can be carried out in such a way as to ensure its presence in all germinal cells and somatic cells of the target mammal. The presence of abnormal DNA of the invention in the germinal cells of the transgenic animal means that all subsequent generations of the transgenic animal will have the abnormal DNA of the invention in all their germinal cells and somatic cells. The offspring of animals of this line inheriting the exogenous DNA of the invention will have the abnormal DNA of the present invention in all their germinal cells and somatic cells. Homozygous animals of both sexes having the DNA in both homologous chromosomes can be mated and bred in such a way as to ensure all offspring have the DNA.

Non-human mammals with abnormal DNA of the present invention are characterized by high expression of the abnormal DNA, and may ultimately develop disorders involving functional inactivation of polypeptides of the present invention as a result of the inhibition of the function of normal endogenous DNA, making them useful as disease model animals. For example, abnormal transgenic animals of the present invention can be used to elucidate the mechanisms of disorders involving functional inactivation of the polypeptide of the invention, and to study methods for treating such disorders.

As one specific potential use, animals with high expression of the abnormal DNA of the present invention can be used as a model for elucidating the functional inhibition of the normal polypeptide by an abnormal polypeptide of the present invention (dominant negative effect) in disorders involving functional inactivation of polypeptides of the present invention.

Mammals with abnormal exogenous DNA of the invention may also have exacerbated symptoms associated with free polypeptides of the invention, and can thus be used to screen drugs for the treatment of disorders involving functional inactivation of polypeptides of the invention.

Examples of other potential uses of the above two types of transgenic animals include:

(1) their use as sources of cells for tissue culture;
(2) analysis of the relationship to polypeptides which are specifically expressed or activated by polypeptides of the invention, based on direct analysis of DNA or RNA in tissue of transgenic mammals, or analysis of the composition of polypeptides expressed by the DNA;
(3) culture of cells from tissue containing the DNA by standard tissue culturing techniques for use in research on the function of cells from tissue which is generally not amenable to culture;
(4) Screening of agents which enhance cell function by using cells described in (3) above; and
(5) the isolation and purification of variant polypeptides in the present invention, and the preparation of their antibodies.

Transgenic animals of the present invention can also be used to study the clinical symptoms of diseases associated with polypeptides of the invention, including disorders involving functional inactivation of polypeptides of the invention, in order to obtain more detailed pathological findings in various organs in models of diseases associated with polypeptides of the invention, with the potential for contributing to the development of novel methods of treatment, as well as research on and treatment of secondary diseases caused by such diseases.

Furthermore, various organs can be excised from transgenic animals of the invention, homogenized, and treated with a proteolytic enzyme such as trypsin to obtain free transgenic cells which can be cultured or used to establish a cell line of cultured cells. Such materials make useful research materials for studying polypeptides of the invention and elucidating their activity, such as the characterization of cells producing polypeptides of the invention, and the study of their relationship to apoptosis, differentiation, and proliferation, as well as the mechanism of their signal transduction and abnormalities thereof.

The aforementioned testing methods, quantification methods, and the like can also be used to provide a method for efficient and rapid screening of drugs for such diseases in order to develop drugs for the treatment of diseases associated with polypeptides of the invention, including disorders involving functional inactivation of polypeptides of the invention, using transgenic animals of the invention. Transgenic animals of the invention or exogenous DNA expression vectors of the present invention can also be used to study and develop DNA therapy for diseases associated with polypeptides of the invention.

[8] Knockout Animals

The present invention is also intended to provide non-human mammal embryonic stem cells in which DNA of the present invention has been inactivated, and non-human mammals with deficient expression of DNA in the invention.

Specifically, the invention is intended to provide:

(1) non-human mammal embryonic stem cells in which DNA of the present invention has been inactivated;
(2) embryonic stem cells according to (1) above, wherein the inactivation is brought about through the introduction of a DNA reporter gene (such as the *E. coli* β-galactosidase gene);
(3) non-human mammal embryonic stem cells according to (1) above, which are neomycin-resistant;
(4) non-human mammal embryonic stem cells according to (1) above, wherein the non-human mammal is a rodent;
(5) non-human mammalian embryonic stem cells according to (4) above, wherein the rodent is a mouse;
(6) non-human mammals with deficient expression of DNA in the invention, wherein the DNA of the invention has been inactivated;
(7) non-human mammals according to (6) above, wherein the DNA is inactivated through the introduction of a reporter gene (such as the *E. coli* β-galactosidase gene), and the reporter gene can be expressed under the control of a promoter for the DNA of the present invention;
(8) non-human mammals according to (6) above, wherein the non-human mammal is a rodent;
(9) non-human mammals according to (8) above, wherein the rodent is a mouse; and
(10) a method for screening compounds or their salts which promote or inhibit promoter activity on the DNA of the present invention, characterized by the administration of a test compound to a non-human mammal according to (7) above to search for expression of the reporter gene.

Non-human mammal embryonic stem cells in which DNA of the invention has been inactivated refer to non-human mammal embryonic stem cells (ES cells) in which the DNA expression capacity has been inhibited through the artificial addition of mutations to DNA of the invention possessed by such non-human mammals, or such stem cells in which DNA is substantially deprived of the capacity to express polypeptides of the invention as a result of the substantial loss of the activity of polypeptides of the invention encoded by the DNA (sometimes referred to below as knockout DNA of the invention).

The same non-human mammals described above can be used.

Examples of methods for artificially introducing mutations to DNA of the present invention include the deletion of some or all of a DNA sequence, or the insertion or substitution of other DNA, by genetic engineering techniques. Such mutations should be used, for example, to shift the codon reading frame or disrupt promoter or exon functions in order to produce knockout DNA of the invention.

Specific examples of non-human mammal embryonic stem cells in which DNA of the invention has been inactivated (referred to below as DNA-inactivated ES cells of the invention or knockout ES cells of the invention) can be obtained by isolating target DNA of the invention possessed by non-human mammals, inserting a drug resistance gene, such as the neomycin resistance gene or hygromycin resistance gene, or a reporter gene, such as lacZ (β-galactosidase gene) or cat (chloramphenicol acetyl transferase gene), into the exon portions to disrupt the exon function, or inserting a DNA sequence that terminates gene transcription (such as a polyA linker signal) between exons to disable synthesis of complete mRNA, inserting the resulting DNA strand having the DNA sequence thus constructed to disrupt the gene (referred to below as targeting vector) into the chromosomes of an animal by homologous recombination, for example, and analyzing the resulting ES cells by Southern hybridization using probe comprising a DNA sequence on or near the DNA of the invention or by PCR using primers comprising the DNA sequence on the targeting vector and the DNA sequence of another region near the DNA of the invention used to produce the targeting vector, so as to screen for knockout ES cells of the invention.

Examples of original ES cells in which the DNA of the invention is to be inactivated by homologous recombination or the like include those which have already been established such as the above and new lines established according to the known method of Evans and Kaufman. For example, ES cells of the 129 line are generally used at present in the case of mouse ES cells, but since the immunological background is not very well known, it can be more beneficial to use lines which have been established using C57BL/6 or $BDF_1$ mice ($F_1$ of C57BL/6 and DBA/2), a strain obtained by improving the low fertility of the C57BL/6 breed through hybridization with DBA/2, for example, in order to obtain ES cells which are from a pure line and have a known immunological background. In addition to the advantages of fertility and healthy eggs, $BDF_1$ mice have the background of C57/BL/6 mice, so a benefit of ES cells obtained using them is that the immunological background can be converted to that of C57BL/6 mice by being back-crossed with C57BL/6 mice when producing disease model mice.

Blastocysts are commonly used 3.5 days after fertilization when establishing an ES cell line, but large numbers of early embryos can otherwise be efficiently obtained by culturing 8-cell stage embryos until the blastocyst stage.

Although ES cells of either sex may be used, male ES cells are usually more convenient for producing germ line chimeras. The sexes should also be distinguished as soon as possible in order to minimize the complexity of the culture procedures.

An example of a method for sexing ES cells is to amplify and detect the sex-determining region on the Y chromosome by PCR. Approximately $10^6$ cells are required in conventional karyotype analysis, whereas only about 1 colony of ES cells (about 50) is needed in this method. This sexing method thus permits the primary selection of ES cells in the initial stages of culture, and also allows male cells to be selected at an early stage, considerably simplifying the early stages of culture.

Secondary selection can be carried out through the verification of the number of chromosomes by G-banding, for example. The number of chromosomes of the resulting ES cells should be 100% of the normal number, but in cases where this is complicated by the physical operations or the like involved in establishing a line, the gene of the ES cell should be knocked out and recloned to normal cells (such as cells with a chromosome number 2n = 40 in mice).

The embryonic stem cell line thus established is generally characterized by extremely good growth, but must be subcultured with extreme care because the ontogenic capacity tends to be lost. For example, the cell line should be cultured on suitable feeder cells such as STO fibroblasts in the presence of LIF (1 to 10,000 U/ml) in a carbon dioxide culture vessel (preferably 5% $CO_2$ and 95% air, or 5% oxygen, 5% $CO_2$, and 90% air) at about 37°C. During subculture, the cells should be treated, for example, with trypsin/EDTA solution (usually 0.001 to 0.5% trypsin/0.1 to 5 mM EDTA, and preferably about 0.1% trypsin/l mM EDTA) to produce single cells, which are then inoculated onto fresh feeder cells. Such subculture is usually performed every 1 to 3 days, but the cells should be monitored in the meantime, and any morphologically abnormal cells that are discovered should be discarded.

ES cells can be allowed to differentiate into various types of cells, such as those of the longus capitis muscle, visceral muscles, or cardiac muscle, through monolayer culture to high density under suitable conditions, or through suspension culture until the formation of a cell mass (M. J. Evans & M. H. Kaufman, *Nature,* **292**, 154 (1981); G. R. Martin, *Proceedings of National Academy of Science USA,* **78**, 7634 (1981); and T. C. Doetschman et al., *Journal of Embryology and Experimental Morphology,* **87**, 27 (1985)). Cells with deficient expression of DNA of the present invention obtained upon the differentiation of the ES cells of the invention are useful for in vitro cytobiological analysis of polypeptides of the present invention and receptor proteins of the invention.

Non-human mammals with deficient expression of DNA of the present invention can be distinguished from normal animals by assaying the levels of mRNA in the animals in the usual manner and by indirectly comparing the levels of expression.

Examples of such non-human mammals include those noted above.

Non-human mammals with deficient expression of DNA of the present invention can be produced by knocking out DNA of the invention through homologous recombination, where a targeting vector as described above is introduced to mouse embryonic stem cells or mouse oocytes, and as a result of its introduction, the DNA sequence of the targeting vector with inactivated DNA of the invention replaces DNA of the invention on the chromosomes of the mouse embryonic stem cells or mouse oocytes through genetic homologous recombination.

Cells in which DNA of the invention has been knocked out can be determined by Southern hybridization analysis with probe comprising a DNA sequence on or near DNA of the invention, or by PCR with primers comprising the DNA sequence on the targeting vector and a DNA sequence in a nearby region other than the mouse DNA of the invention used in the targeting vector. When non-human mammal embryonic stem cells are used, a cell line in which DNA of the present invention has been inactivated by homologous recombination can be cloned, the cells can be injected into non-human mammal embryos or blastocysts at a suitable stage, such as the 8-cell stage, and the resulting chimeric embryos can be transplanted to the uterus of a surrogate non-human mammal. The resulting animal will be a chimeric animal comprising both cells with the normal DNA locus of the present invention and cells with artificially mutated DNA of the present invention.

When some germ cells of the chimeric animal have the mutated DNA locus of the present invention, chimeric individuals can be mated with normal individuals, and individuals in which all tissue comprises cells with the artificially mutated DNA locus of the invention can be selected from the group of individuals resulting from the above mating, on the basis of coat color, for example. The resulting individuals are usually characterized by deficient heterogeneous expression of polypeptides of the invention. The mating of individuals with deficient heterogeneous expression of polypeptides of the invention or receptor proteins of the invention can produce individuals with deficient homogeneous expression of the invention or receptor proteins of the invention.

When oocytes are used, DNA solution can be injected by microinjection into the nucleus of the oocytes to produce transgenic non-human mammals with the targeting vector introduced into the chromosomes, and those animals with mutations in the DNA locus of the present invention resulting from homologous recombination can be selected in comparison to the above transgenic non-human mammals.

Individuals in which DNA of the present invention has been knocked out can be mated to verify that the resulting individuals also have the DNA knocked out, and can be bred and raised under the usual breeding conditions.

The germ line should be obtained and maintained in the usual manner. Specifically, animals of both sexes conserving the inactivated DNA can be mated to obtain homozygous animals with the inactivated DNA in both chromosomes. The resulting homozygous animals can be efficiently obtained when bred under conditions giving 1 normal individual and several homozygotes per dam. Heterozygous animals of both sexes can be mated to breed and raise heterozygous and homozygous animals with the inactivated DNA.

Non-human mammal embryonic stem cells in which DNA of the invention has been inactivated are extremely useful for producing non-human mammals with deficient expression of DNA of the invention.

Because non-human mammals with deficient expression of DNA of the invention lack a variety of physiologically active substances which can be induced by polypeptides of the invention or receptor proteins of the invention, such animals can serve as models of disease caused by inactivation of the physiological activity of polypeptides of the invention or receptor proteins of the invention, and can thus be useful to research the causes of such diseases and to study therapies for them.

[8a] Method for Screening Compounds With Effects in the Treatment and Prevention of Diseases Caused by Missing or Damaged DNA of the Invention

Non-human mammals with deficient expression of DNA in the invention can be used to screen for compounds having effects in the treatment and prevention of diseases caused by missing or damaged DNA of the invention.

Specifically, the present invention is intended to provide a method for screening compounds or their salts having prophylactic or therapeutic effect on diseases caused by missing or damaged DNA of the invention, characterized by the administration of test compounds to non-human mammals with deficient expression of DNA in the invention, and the observation and measurement of resulting changes in the animals.

The non-human mammals with deficient expression of DNA in the invention used in the above screening method are the same as the animals described above.

Examples of test compounds include peptides, proteins, non-peptide compounds, synthetic compounds, fermented products, cell extracts, plant extracts, animal tissue extract, and plasma. Such compounds may be novel compounds or known compounds.

Specifically, non-human mammals with deficient expression of DNA in the invention can be treated with test compounds to test the therapeutic and prophylactic effects of such compounds based on changes such as in the various animal organs, tissue, or symptoms of disease compared to untreated control animals.

Methods for treating test animals with test compounds include oral administration or intravenous injection, which can be selected according to the condition of the test animals, the characteristics of the test compound, and the like. The compound dosage can be selected depending on the method of administration, the characteristics of the test compound, and the like.

Glucose loading can be performed on non-human mammals with deficient expression of DNA of the invention, test compounds can be given before and after glucose loading, and the animals can be monitored for changes over time in blood glucose levels, body weight, and the like when screening for compounds with therapeutic and prophylactic effect on diseases such as hypertension, autoimmune diseases, cardiac failure, cataracts, glaucoma, acute bacterial meningitis, acute myocardial infarction, acute pancreatitis, acute viral encephalitis, adult respiratory distress syndrome, alcoholic hepatitis, Alzheimer's disease, asthma, atherosclerosis, atopic dermatitis, bacterial pneumonia, bladder carcinoma, bone fractures, breast cancer, hyperexia, adphagia, burn treatment, uterine cancer, chronic lymphatic leukemia, chronic myeloid leukemia, chronic pancreatitis, hepatic cirrhosis, colon cancer (colorectal cancer), Crohn's disease, dementia, diabetic complications, diabetic nephropathy, diabetic neuropathy, diabetic retinopathy, gastritis, *Helicobacter pylori* infection, hepatic insufficiency, hepatitis A, hepatitis B, hepatitis C, hepatitis, Herpes simplex, Herpes zoster, Hodgkin's disease, AIDS, human papilloma viral infections, hypercalcemia, hypercholesterolemia, hypertriglyceridemia, hyperlipemia, infections, influenza viral infections, insulin-dependent diabetes (type I), invasive Staphylococcus infection, malignant melanoma, metastatic cancer, multiple myeloma, allergic rhinitis, nephritis, non-Hodgkin's lymphoma, non-insulin-dependent diabetes (type II), non-small cell lung cancer, organ transplants, osteoarthropy, osteomalacia, osteopenia, osteoporosis, ovarian cancer, bone Paget disease, peptic ulcers, peripheral blood vessel disease, prostate cancer, esophageal reflux, renal insufficiency, rheumatoid arthritis, schizophrenia, sepsis, septic shock, serious systemic mycosis, small-cell lung cancer, spinal injuries, stomach cancer, systemic lupus erythematosus, transient ischemic attack, tuberculosis, valvular heart disease, vascular/multi-infarct dementia, trauma therapy, insomnia, arthritis, insufficiency of pituitary hormone secretion, pollakiuria, uremia, and neurodegenerative diseases.

When test compounds are administered to test animals in the screening method, test compounds can be selected as compounds having therapeutic and prophylactic effect on the aforementioned diseases whenever the animal blood glucose level has been reduced at least about 10%, preferably at least about 30%, and even more preferably at least about 50%.

Because compounds obtained using such a screening method are compounds which have been selected from the aforementioned test compounds and which have therapeutic and prophylactic effect on diseases induced by missing or damaged polypeptides of the invention, they can be used as safe drugs with low toxicity for the treatment and prevention of such diseases. Compounds derived from compounds obtained in the above screening can similarly be used.

Compounds obtained by the screening method may be formed into salts. Examples of salts of such compounds include salts with pharmaceutically acceptable acids (such as inorganic and organic acids) or bases (such as alkali metals). Pharmaceutically acceptable acid salts are particularly preferred. Examples of such salts include salts with inorganic acids (such as hydrochloric acid, phosphoric acid, hydrobromic acid, and sulfuric acid), or salts with organic acids (such as acetic acid, formic acid, propionic acid, fumaric acid, maleic acid, succinic acid, tartaric acid, citric acid, malic acid, oxalic acid, benzoic acid, methanesulfonic acid, and benzenesulfonic acid).

Therapeutic drugs containing such compounds or their salts obtained in the screening method can be produced in the same manner as drugs containing polypeptides of the invention as described above.

The resulting preparations are safe and have low toxicity, and can thus be administered, for example, to human or mammals (such as rats, mice, guinea pigs, rabbits, sheep, pigs, cows, horses, cats, dogs, and monkeys).

The dosage of such compounds or their salts will vary depending on the target disease, purpose of administration, route of administration or the like, but the daily adult oral dosage of such compounds may generally range from about 0.1 to 100 mg, preferably from about 1.0 to 50 mg, and even more preferably from about 1.0 to 20 mg, in terms of compound (per 60 kg body weight). The single parenteral dose of such compounds will also vary depending on the purpose of administration, target disease, and the like, but in the form of an intravenous injection for adults, for example, the daily dosage of such compounds may usually range from about 0.01 to about 30 mg, preferably about 0.1 to about 20 mg, and even more preferably about 0.1 to about 10 mg, in terms of the compound (per 60 kg body weight), given by intravenous injection. Doses for animals can also be given as calculated per 60 kg body weight.

[8b] Method for Screening Compounds That Promote or Inhibit Activity of Promoters on DNA of the Invention

The present invention is also intended to provide a method for screening compounds or their salts that promote or inhibit the activity of promoters on DNA of the invention, characterized by the administration of test compounds to non-human mammals with deficient expression of DNA of the present invention and the detection of the expression of reporter genes.

The non-human mammals with deficient expression of DNA of the invention used in the aforementioned screening method are non-human mammals with deficient expression of the DNA of the invention in which the DNA of the invention has been inactivated through the introduction of a reporter gene, and the reporter gene can be expressed under the control of a promoter for the DNA of the invention.

Examples of such test compounds include the same ones described above.

Examples of reporter genes include the same ones described above. Suitable examples include the β-galactosidase gene (lacZ), soluble alkaline phosphatase gene, and luciferase gene.

Because the reporter gene is under the control of a promoter for DNA of the invention in non-human mammals with deficient expression of the DNA of the invention in which the DNA of the invention has been replaced by the reporter gene, the promoter activity can be detected by tracing the expression of substances encoded by the reporter gene.

For example, when a portion of the DNA region coding for a polypeptide of the invention is replaced with the *E. coli* β-galactosidase gene (lacZ), β-galactosidase will be expressed instead of the polypeptide of the invention in the tissue where the polypeptide of the invention is intrinsically expressed. A reagent serving as the substrate for the β-galactosidase such as 5-bromo-4-chloro-3-indolyl-β-galactopyranoside (X-gal) can thus be used for staining, allowing the expression of the polypeptide of the invention to be readily monitored in the body of the animal. Specifically, a polypeptide-deficient mouse of the invention or tissue slices therefrom can be immobilized with glutaraldehyde and washed with phosphate-buffered saline (PBS), a reaction can then be brought about for about 30 minutes to 1 hour around ambient temperature or 37°C with stain solution containing X-gal, and the tissue samples can then be washed with 1 mM EDTA/PBS solution to stop the (β-galactosidase reaction and observe the staining. mRNA coding for lacZ may be detected in the usual manner.

Compounds obtained using the aforementioned compounds or salts are compounds that have been selected from the aforementioned test compounds and that promote or inhibit promoter activity on DNA of the invention.

Compounds obtained in the screening method may be formed into salts. Examples of salts of such compounds include salts with pharmaceutically acceptable acids (such as inorganic acids) or bases (such as organic acids), and especially pharmaceutically acceptable acid salts. Examples of such salts include salts with inorganic acids (such as hydrochloric acid, phosphoric acid, hydrobromic acid, and sulfuric acid), or salts with organic acids (such as acetic acid, formic acid, propionic acid, fumaric acid, maleic acid, succinic acid, tartaric acid, citric acid, malic acid, oxalic acid, benzoic acid, methanesulfonic acid, and benzenesulfonic acid).

Since compounds or their salts that promote or inhibit promoter activity on DNA in the invention can promote or inhibit the expression of polypeptides in the invention and can promote or inhibit polypeptide functions, they can be useful as safe agents with low toxicity for the treatment and prevention of diseases such as hypertension, autoimmune diseases, cardiac failure, cataracts, glaucoma, acute bacterial meningitis, acute myocardial infarction, acute pancreatitis, acute viral encephalitis, adult respiratory distress syndrome, alcoholic hepatitis, Alzheimer's disease, asthma, atherosclerosis, atopic dermatitis, bacterial pneumonia, bladder carcinoma, bone fractures, breast cancer, hyperexia, adphagia, burn treatment, uterine cancer, chronic lymphatic leukemia, chronic myeloid leukemia, chronic pancreatitis, hepatic cirrhosis, colon cancer (colorectal cancer), Crohn's disease, dementia, diabetic complications, diabetic nephropathy, diabetic neuropathy, diabetic retinopathy, gastritis, *Helicobacter pylori* infection, hepatic insufficiency, hepatitis A, hepatitis B, hepatitis C, hepatitis, Herpes simplex, Herpes zoster, Hodgkin's disease, AIDS, human papilloma viral infections, hypercalcemia, hypercholesterolemia, hypertriglyceridemia, hyperlipemia, infections, influenza viral infections, insulin-dependent diabetes (type I), invasive Staphylococcus infection, malignant melanoma, metastatic cancer, multiple myeloma, allergic rhinitis, nephritis, non-Hodgkin's lymphoma, non-insulin-dependent diabetes (type II), non-small cell lung cancer, organ transplants, osteoarthropy, osteomalacia, osteopenia, osteoporosis, ovarian cancer, bone Paget disease, peptic ulcers, peripheral blood vessel disease, prostate cancer, esophageal reflux, renal insufficiency, rheumatoid arthritis, schizophrenia, sepsis, septic shock, serious systemic mycosis, small-cell lung cancer, spinal injuries, stomach cancer, systemic lupus erythematosus, transient ischemic attack, tuberculosis, valvular heart disease, vascular/multi-infarct dementia, trauma therapy, insomnia, arthritis, insufficiency of pituitary hormone secretion, pollakiuria, uremia, and neurodegenerative diseases.

Compounds derived from compounds obtained in the aforementioned screening can be the same as those described above.

Therapeutic drugs containing compounds or their salts obtained in the aforementioned screening can be produced in the same manner as drugs containing the aforementioned polypeptides or salts.

The resulting preparations are safe and have low toxicity, and can thus be administered, for example, to human or mammals (such as rats, mice, guinea pigs, rabbits, sheep, pigs, cows, horses, cats, dogs, and monkeys).

The dosage of such compounds or their salts will vary depending on the target disease, purpose of administration, route of administration or the like, but the daily adult oral dosage of compounds promoting promoter activity on DNA of the invention may generally range from about 0.1 to 100 mg, preferably from about 1.0 to 50 mg, and even more preferably from about 1.0 to 20 mg, in terms of compound (per 60 kg body weight). The single parenteral dose of such compounds will also vary depending on the purpose of administration, target disease, and the like, but in the form of an intravenous injection for adults, for example, the daily dosage of such compounds may usually range from about 0.01 to about 30 mg, preferably about 0.1 to about 20 mg, and even more preferably about 0.1 to about 10 mg, in terms of the compound (per 60 kg body weight), given by intravenous injection. Doses for other animals can also be given as calculated per 60 kg body weight.

The daily adult oral dosage of compounds inhibiting promoter activity on DNA of the invention may generally range from about 0.1 to 100 mg, preferably from about 1.0 to 50 mg, and even more preferably from about 1.0 to 20 mg, in terms of compound (per 60 kg body weight). The single parenteral dose of such compounds will also vary depending on the purpose of administration, target disease, and the like, but in the form of an intravenous injection for adults, for example, the daily dosage of such compounds may usually range from about 0.01 to about 30 mg, preferably about 0.1 to about 20 mg, and even more preferably about 0.1 to about 10 mg, in terms of the compound (per 60 kg body weight), given by intravenous injection. Doses for other animals can also be given as calculated per 60 kg body weight.

In this way, non-human mammals with deficient expression of DNA in the invention may be extremely useful for screening compounds or their salts that promote or inhibit promoter activity on DNA in the invention, and can contribute significantly to research on the causes of various diseases resulting from deficient expression of DNA in the invention, and to development of therapeutic and prophylactic drugs.

DNA containing a promoter region for polypeptides in the invention can be used to ligate genes coding for various proteins downstream of the promoter for injection into the oocytes of animals to produce what are referred to as transgenic animals (animals to which the gene has been transferred), so as to enable specific synthesis of such polypeptides to study their in vivo activity. A suitable reporter gene can be ligated to the aforementioned promoter region, and an expression cell line can be established and thus used as a screening system for low molecular weight compounds with activity of specifically promoting or inhibiting the in vivo production of polypeptides of the invention.

The abbreviations for bases, amino acids, and the like in the Specification and drawings are based on the abbreviations authorized by the IUPAC-IUB Commission on Biochemical Nomenclature, or other abbreviations commonly used in the field, examples of which are given below. Unless otherwise indicated, amino acid optical isomers are the L form.

| | |
|---|---|
| DNA: | deoxyribonucleic acid |
| cDNA: | complementary deoxyribonucleic acid |
| A: | adenine |
| T: | thymine |

G: guanine
C: cytosine
I: inosine
R: adenine (A) or guanine (G)
Y: thymine (T) or cytosine (C)
M: adenine (A) or cytosine (C)
K: guanine (G) or thymine (T)
S: guanine (G) or cytosine (C)
W: adenine (A) or thymine (T)
B: guanine (G), guanine (G) or thymine (T)
D: adenine (A), guanine (G) or thymine (T)
V: adenine (A), guanine (G) or cytosine (C)
N: adenine (A), guanine (G), cytosine (C), or thymine (T) or another unknown base
RNA: ribonucleic acid
mRNA: messenger ribonucleic acid
dATP: deoxyadenosine triphosphate
dTTP: deoxythymidine triphosphate
dGTP: deoxyguanosine triphosphate
dCTP: deoxycytidine triphosphate
ATP: adenosine triphosphate
EDTA: ethylenediaminetetracetic acid
SDS: sodium dodecylsulfate
BHA: benzhydrylamine
pMBHA: p-methylbenzhydrylamine
Tos: p-toluenesulfonyl
Bzl: benzyl
Bom: benzyloxymethyl
Boc: t-butyloxycarbonyl
DCM: dichloromethane
HOBt: 1-hydroxybenztriazole
DCC: N,N'-dicyclohexylcarbodiimide
TFA: trifluoroacetic acid
DIEA: diisopropylethylamine
CHO: formyl
$Cl_2$-Bzl: 2,6-dichlorobenzyl
Z: benzyloxycarbonyl
Cl-Z: 2-chlorobenzyloxycarbonyl
Br-Z: 2-bromobenzyloxycarbonyl
DNP: dinitrophenyl
Trt: trityl
Bum: tert-butoxymethyl
Fmoc: N-9-fluorenylmethoxycarbonyl
HOOBt: 3,4-dihydro-3-hydroxy-4-oxo-1,2,3-benzotriazine
HONB: 1-hydroxy-5-norbornene-2,3-dicarboximide

Gly: glycine
Ala: alanine
Val: valine
Leu: leucine
Ile: isoleucine
Ser: serine
Thr: threonine
Cys: cystine
Met: methionine
Glu: glutamic acid
Asp: aspartic acid
Lys: lysine
Arg: arginine

His: histidine
Phe: phenylalanine
Tyr: tyrosine
Trp: tryptophan
Pro: proline
Asn: asparagine
Gln: glutamine
pGlu: pyroglutamic acid

The SEQ ID NOS. in the Sequence Listing of the present application indicate the following sequences.

SEQ ID NO. 1:
The amino acid sequence for the polypeptide (human type) of the invention obtained in Example 1 below;
SEQ ID NO. 2:
The base sequence of DNA coding for the polypeptide of the invention having the amino acid sequence represented by SEQ ID NO. 1;
SEQ ID NO. 3:
The base sequence of Primer LF2 used in Example 1 below;
SEQ ID NO. 4:
The base sequence of Primer LR1 used in Example 1 below;
SEQ ID NO. 5:
The amino acid sequence having the deletion of the residues from the position 1 through 24 from the N terminal, presumably a signal sequence, in the amino acid sequence of the polypeptide of the invention obtained in Example 1 below;
SEQ ID NO. 6:
The base sequence of DNA coding for the polypeptide of the invention having the amino acid sequence represented by SEQ ID NO. 5;
SEQ ID NO. 7:
The amino acid sequence of the human type alpha subunit;
SEQ ID NO. 8:
The base sequence of DNA coding for the polypeptide of the invention having the amino acid sequence represented by SEQ ID NO. 7;
SEQ ID NO. 9:
The base sequence of Primer r25F2 used in Example 2 below;
SEQ ID NO. 10:
The base sequence of Primer r25R1 used in Example 1 below;
SEQ ID NO. 11:
The amino acid sequence of the polypeptide (rat type) of the invention obtained in Example 2 below;
SEQ ID NO. 12:
The base sequence of DNA coding for the polypeptide of the invention having the amino acid sequence represented by SEQ ID NO. 11;
SEQ ID NO. 13:
The amino acid sequence having the deletion of the residues from the position 1 through 23 from the N terminal, presumably a signal sequence, in the amino acid sequence of the polypeptide of the invention obtained in Example 2 below;
SEQ ID NO. 14:
The base sequence of DNA coding for the polypeptide of the invention having the amino acid sequence represented by SEQ ID NO. 13;
SEQ ID NO. 15:
The base sequence of Primer VHFX1 used in Example 3 below;
SEQ ID NO. 16:
The base sequence of Primer VHRN1 used in Example 3 below.

## EXAMPLES

Examples are given below to illustrate the invention in further detail, but the present invention is not limited by these examples. Gene manipulation using *Escherichia coli* was carried out in accordance with the procedures described in *"Molecular Cloning"*.

The *Escherichia coli* JM109/pTAhGTHL6 having the pTAhGTHL6 plasmid obtained in Example 1 was deposited as FERM BP-7356 at the National Institute of Bioscience and Human Technology (NIBH), Agency of Industrial Science and Technology, Ministry of International Trade and Industry, 1-1-3 Higashi, Tsukuba-shi, Ibaraki-ken, Japan (Postal No. 305-8566) on November 9, 2000, and was also deposited as IFO 16489 at the Institute of Fermentation, Osaka (IFO), 2-17-85 Juso-honmachi, Yodogawa-ku, Osaka-shi, Japan (Postal No. 532-8686) on October 24, 2000.
The *Escherichia coli* JM109/prGON7 having the prGON7 plasmid obtained in Example 2 was deposited as FERM BP-7354 at NIBH on November 9, 2000, and as IFO 16487 at IFO on October 24, 2000.

<u>Example 1</u>: cDNA synthesis from human pituitary poly(A)$^+$ RNA fraction, and isolation of cDNA of the novel hormone VH098489 by RT-PCR

cDNA was synthesized by means of Moloney mouse leukemia virus reverse transcriptase(Gibco BRL) in the attached buffer, to which 1 μg human pituitary poly(A)$^+$ RNA fraction(Clontech) and oligo (dT) primer(Gibco BRL) as primer were added. The reaction product was precipitated with ethanol and then dissolved in 100 μL TE. 0.1 μL of the resulting cDNA was used as template in PCR amplification with the following two synthetic DNAs.

LF2:5'-CGGAAGAGCAGCATGAAGCTGGCATTC-3' (SEQ ID NO. 3)

LR1:5'-CATGTGCTGCTCACACAGGTGGGTCTG-3' (SEQ ID NO. 4)

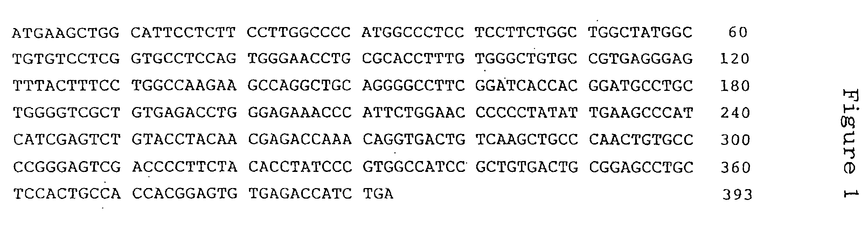

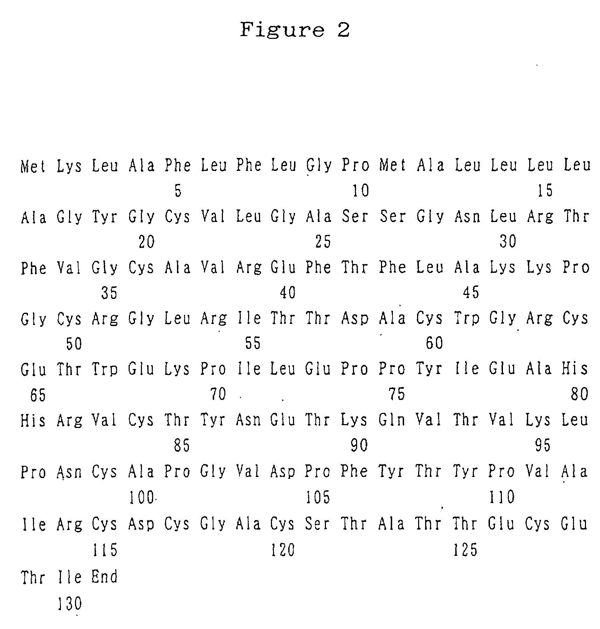

The PCR reaction solution was a total of 25 μL containing 0.1 μL cDNA solution (derived from 1 ng poly(A)+ RNA), 0.5 μL LF2 (10 μM), 0.5 μL LR1 (10 μM), 2.5 μL the attached 10× reaction solution, 2.5 μL dNTP (10 mM), 0.25 μL Ex Taq (Takara), and 18.65 μL distilled water. The reaction solution was subjected to PCR with a Thermal Cycler 9600. Denaturation for 2 min. at 95°C was followed by repeating 35 cycles, where a cycle was 10 seconds at 98°C, 10 seconds at 65°C, and 20 seconds at 72°C. A portion of the PCR product was used in electrophoresis to check that the approximately 450 bp PCR product had been amplified, and the PCR product was then purified using a Quiagen PCR Purification Kit and the DNA sequence was directly determined to have the sequence shown in Figure 1. Figure 2 shows the amino acid sequence deduced on the basis of the DNA sequence in Figure 1. A typical highly hydrophobic signal sequence was found in the N terminal region of the deduced amino acid sequence, and the protein was assumed to be secreted upon cleavage around the Gly at position 24 and the Ala at position 25. The possibility of sugar chain linkage at the Asn at position 87 was expected on the basis of the sequence. Based on comparisons with other hormones, disulfide bonds were assumed between the cysteine residues at positions 36-84, 50-99, 60-115, 64-117, and 120-127. The absence of cysteines capable of disulfide bonds, which are considered essential for association with the alpha subunit in LH, FSH, TSH, and the like, was particularly interesting, suggesting that this novel hormone VH098489 is capable of work alone without the subunit structure formed. Study of the homology of the resulting sequence revealed high homology with known pituitary hormones, particularly the beta subunit of LH, FSH, TSH, and the like (Figure 3). The approximately 450 bp PCR product was inserted into the pCR2.1 Topo cloning vector according to the manual in the TA Cloning Kit(Invitrogen), and the vector was introduced into *E. coli* JM109, giving the *E. coli* JM109/pTAhGTHL6 transformant.

<u>Example 2</u>: Isolation of rat type cDNA of the novel hormone VH098489 derived from rat thymus

cDNA synthesized from rat thymus poly(A)$^+$ RNA was used as template to obtain cDNA of the rat type of novel hormone VH098489 by PCR.
The following primers were synthesized for the upstream region of the initiation codon and downstream region of the termination codon:

r25F2:5'-AGGCAGCCTGATAACAGAAGGGAGAG-3' (SEQ ID NO. 9)

r25R1:5'-CTTGGGCCACCAGCCATGACTGTGCT-3' (SEQ ID NO. 10)

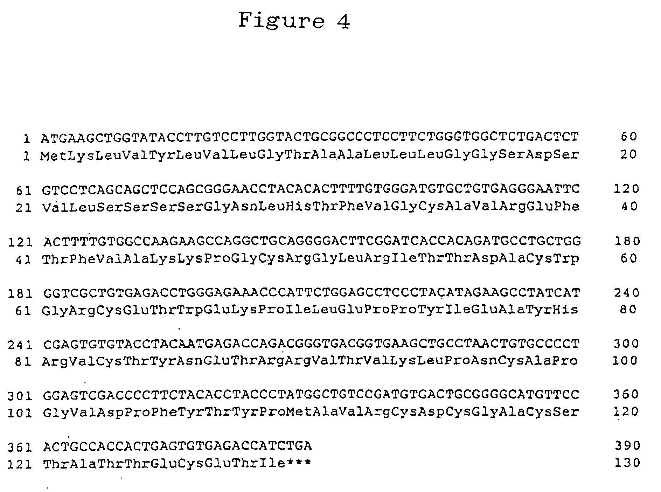

The PCR was carried out using cDNA as template, which was synthesized with the use of Super Script II reverse transcriptase(Gibco BRL) and random 7 mer (Gibco BRL) from rat thymus poly(A)$^+$ RNA. The PCR reaction solution was a total of 25 μL containing 2.5 μL the attached buffer, 200 pM each of the primers and 0.1 mM each of dNTP with Advantage 2 Polymerase(Clontech) as the DNA polymerase, and 34 cycles were run, where a cycle was 10 seconds at 98°C and 45 seconds at 68°C. The PCR reaction product was electrophoresed on 2% agarose, and bands were detected by ethidium bromide staining. The approximately 540 bp band was purified using a QIA Quick Gel Extraction Kit(Qiagen) and inserted into the pCR2.1 Topo cloning vector according to the manual in the TA Cloning Kit(Invitrogen), and the vector was introduced into *E. coli* JM109, giving the *E. coli* JM109/prGON7 transformant. The sequence inserted into the prGON7 plasmid was determined (SEQ ID NO. 12). Figure 4 shows the base sequence and the deduced amino acid sequence(SEQ ID NO. 11) of the novel rat type hormone VH098489 encoded by the base sequence.

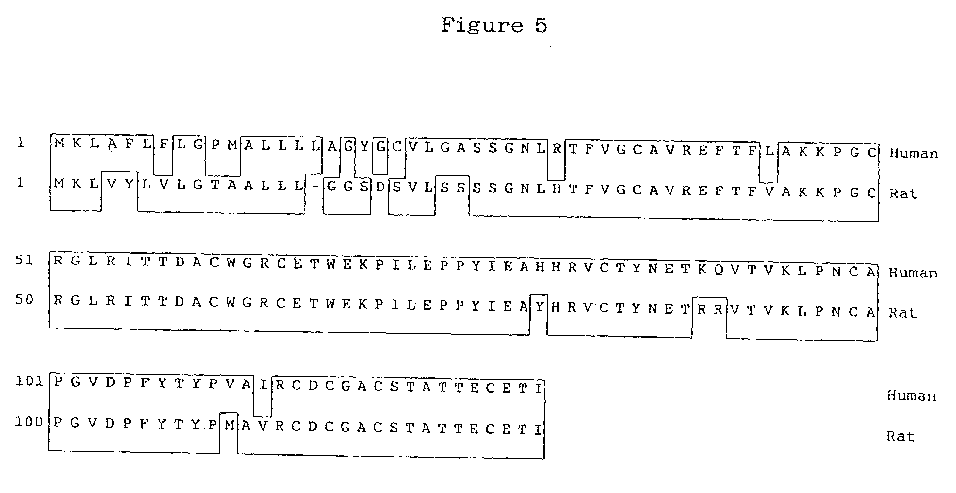

Figure 5 shows a comparison of the rat type sequence with the human type sequence obtained in Example 1.

Example 3: Preparation of CHO cells expressing human type novel hormone VH098489

CHO cells expressing the human type novel hormone VH098489 were prepared in the following manner. The following two synthetic DNAs were synthesized on the basis of the GTHL sequence.

VHFX1: 5'-CTCGAGAGCAGCATGAAGCTGGCATTCCTCT-3' (SEQ ID NO. 15)

VHRN1: 5'-GCTAGCGGCCTCAGATGGTCTCACACTCCGT-3' (SEQ ID NO. 16)

PCR was carried out using these synthetic DNAs and the pTAhGTHL6 plasmid contained in the deposited *Escherichia coli* JM109/pTAhGTHL6 prepared in Example 1 as template. The PCR reaction solution was a total of 50 μL containing 1 μL (1 ng) plasmid solution, 1 μL VHFX1 (10 μM), 1 μL VHRN1 (10 μM), 5 μL the attached 10× reaction solution, 5 μL dNTP (10 mM), 1 μL Ex Taq (Takara), and 36 μL distilled water. The reaction solution was subjected to PCR with a Thermal Cycler 9600. Denaturation for 2 min. at 95°C was followed by repeating 20 cycles, where a cycle was 10 seconds at 98°C, 10 seconds at 65°C, and 30 seconds at 72°C. A portion of the PCR product was used in electrophoresis to check that the approximately 0.5 kb PCR product had been amplified, and the PCR product recovered from the gel was then subcloned to *E. coli* using a TA cloning kit (Invitrogen). The plasmid was extracted using a plasmid extractor(Kurabo) from the subcloned *E. coli,* and the base sequence of the inserted fragment was determined to verify that the sequence was the same human type novel hormone VH098489 cDNA as in Example 1. The plasmid is then digested with the restriction enzymes XhoI and NheI, giving an approximately 0.5 k cDNA fragment of the human type novel hormone VH098489. pAKKO-111H, an expression vector for animal cells, was digested with the multi-cloning site. restriction enzymes SalI and NheI and electrophoresed to recover the vector portion. The resulting expression vector and cDNA fragment of the human type novel hormone VH098489 were ligated and then used to transform *E. coli* JM109, giving *E. coli* JM109/pAKKOGTHL.

The *E. coli* JM109/pAKKOGTHL transformant was cultured for mass production of the DNA of the pAKKOGTHL plasmid.

20 μg of the plasmid DNA was dissolved in 1 mL physiological saline (PBS), then added into gene transfer vials(Wako Pure Chemicals), and vigorously stirred using a vortex mixer to form liposomes containing the DNA. 1 to $2 \times 10^6$ CHO dhfr cells were on cell culture petri dishes 35 mm in diameter. After 20 hour incubation, the culture medium was replaced with fresh medium. Liposome solution in an amount (25 μL) corresponding to 0.5 μg DNA was droped to each dish, and incubated for 16 hours for the introduction of the plasmid DNA. Then, the medium was replaced with fresh medium, followed by cultivation for a day. Furhter, the medium was replaced with the selection medium, follwed by cutivation for 3 days. Finally, the cells, dispersed with trypsin digestion, were inoculated at low density in the selection media (deoxyribonucleoside- and ribonucleoside-free alpha-minimum essential medium supplemented with 10% dialyzed bovine serum) to select a transformant. The only transformant can grow in the selection media, and thus the CHO-GTHL cell line was established through repeated selection by subculturing. Clones with high expression of the human type novel hormone VH098489 were selected by repeating by turns the cell cloning and cultivation in the stepwise increasing concentration of methotrexate(Sigma). The assay of productivity and the selection of highly productive clones were carried out by EIA below at each step.

Example 4: Antiserum against the novel hormone VH098489

Antibodies against the novel hormone VH098489 were prepared in the following manner.

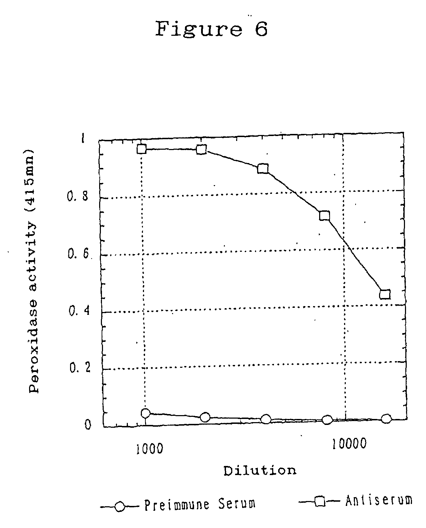

The antigen was ASSGNLRTFVGC (GTN1), comprising the sequence ASSGNLRTFVG immediately after the secretion signal sequence of the novel hormone VH098489 and a cysteine added to the N terminal. Rabbits were immunized with GTN1 conjugated to KLH in the usual manner, giving antiserum against GTN1. Increase in the antibody titer was checked in the usual manner by solid phase EIA using plates coated with OVA-conjugated GTN1 and the horseradish peroxidase-labeled anti-rabbit IgG antibody, giving antiserum with the higher antibody titer shown in Figure 6, as compared to serum prior to immunization.

Example 5: Detection of expression of human type novel hormone VH098489

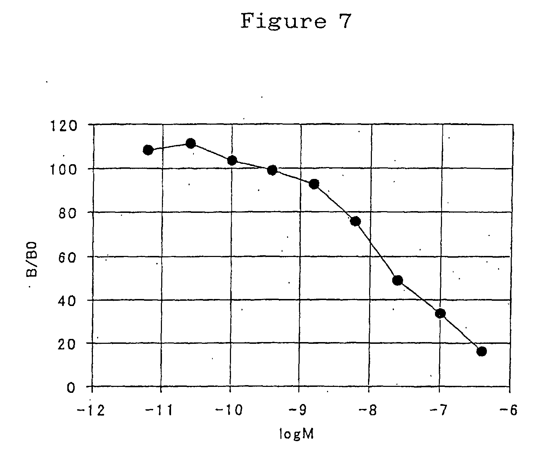

The aforementioned CHO-GTHL cells were cultured under serum-free conditions, and the supernatant was collected and concentrated by ultrafiltration. The concentrated supernatant was dissolved in SDS-PAGE sample buffer, thermally denatured, and then subjected to SDS-PAGE on 16% gel. The proteins separated by electrophoresis were electrically transferred to a nitrocellulose membrane. The membrane was treated with Block Ace(Dainippon Pharmaceutical Co.) to prevent nonspecific binding, and then was incubated with the aforementioned antiserum diluted as needed with PBS containing of 10% Block Ace and 0.1% Tween-20(Sigma). The nitrocellulose membrane was then thoroughly washed and incubated further with the horseradish peroxidase-labeled anti-rabbit IgG antibody, and the unbound labeled antibody was washed off. The human type novel hormone VH098489 transferred to the membrane was detected by coloring reaction with the horseradish peroxidase (Fig. 7).

Example 6: Construction of EIA assay system for novel hormone VH098489

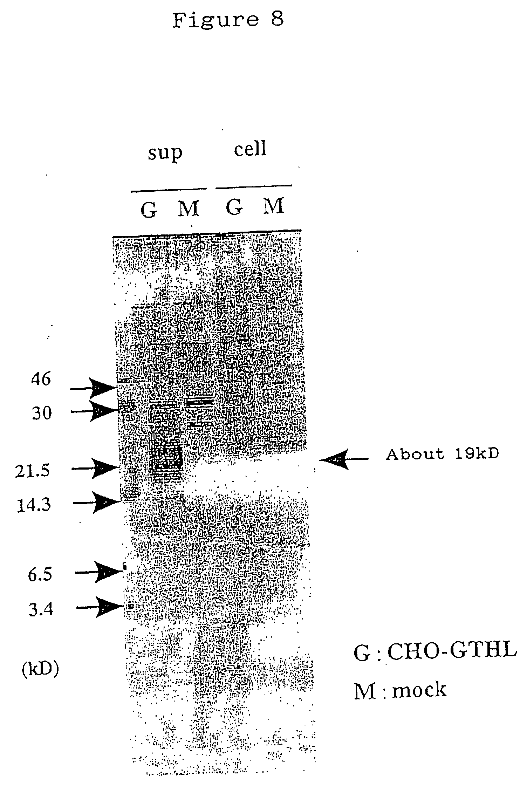

The aforementioned anti-GTN1 antiserum was treated with ammonium sulfate at 50% concentration, and the precipitated fraction was dissolved in PBS. An antigen column was prepared by conjugating GTN1 peptide to a commercially available column (HiTrap NHS-activated, Pharmacia), and the antibody was purified in accordance with the accompanying manual. The GTN1 peptide was conjugated to horseradish peroxidase using maleimide according with standard method, unlabeled peptide was removed by gel filtration to obtain the labeled GTN1. Anti-rabbit IgFc was allowed to bind to 96-well plates for EIA, and then incubated with PBS containing 25% Block Ace to prevent nonspecific binding. After incubation with the purified antibody, and the standard(GTN1 peptide) or a sample at 4°C overnight, the labeled GTN1 was added to the mixture and further incubated at room temperature. The assay buffer was PBS containing 10% Block Ace and 0.1% Tween-20. After the reaction, the plates were thoroughly washed, and the label bound to the plate was detected by coloring reaction with peroxidase. A calibration curve was prepared (Figure 8) based on an amount of the label bound to the plate (B/B0), and used to calculate the concentration of the novel hormone VH098489 contained in samples.

INDUSTRIAL APPLICABILITY

The polypeptides of the invention have physiological activity associated with anterior pituitary hormones (such as LH, FSH, and TSH), and can thus be used as a drug for the treatment of hypertension, autoimmune diseases, cardiac failure, and the like. The polypeptides of the invention are also useful as a reagent for screening compounds or their salts that promote or inhibit activity of polypeptides of the invention, and the compounds obtained by such screening are promising as an agent for the prevention and treatment of hypertension, autoimmune diseases, cardiac failure, and the like. The antibodies against the polypeptides of the invention are also capable of specifically recognizing the polypeptides of the invention, and can thus be used for quantification and the like of the polypeptides of the invention in samples.

## Claims

1. A polypeptide **characterized by** comprising an amino acid sequence that is the same as or substantially the same as the amino acid sequence represented by SEQ ID NO. 1, or an amide or ester thereof, or a salt thereof.

2. A polypeptide, or an amide or ester thereof, or a salt thereof according to Claim 1, wherein the substantially same amino acid sequence includes the amino acid sequence represented by SEQ ID NO. 11.

3. A polypeptide, or an amide or ester thereof, or a salt thereof according to Claim 1, wherein the substantially same amino acid sequence includes the amino acid sequence represented by SEQ ID NO. 5.

4. A polypeptide, or an amide or ester thereof, or a salt thereof according to Claim 1, wherein the substantially same amino acid sequence includes the amino acid sequence represented by SEQ ID NO. 13.

5. A partial peptide of the polypeptide according to Claim 1, or an amide or ester thereof, or a salt thereof.

6. A DNA comprising a DNA coding for the polypeptide according to Claim 1.

7. A DNA according to Claim 6, comprising the base sequence represented in SEQ ID NO. 2, 6, 12, or 14.

8. A DNA comprising a DNA coding for the partial peptide according to Claim 5.

9. A recombinant vector comprising the DNA according to Claim 6 or 8.

**10.** A transformant which is transformed with the recombinant vector according to Claim 9.

**11.** A method for producing a polypeptide, or an amide or ester thereof, or a salt thereof according to Claim 1, or a partial peptide, or an amide or ester thereof, or a salt thereof according to Claim 5, **characterized by** culturing the transformant according to Claim 10 to allow the production and accumulation of the polypeptide according to Claim 1 or the partial peptide according to Claim 5.

**12.** An antibody against a polypeptide, or an amide or ester thereof, or a salt thereof according to Claim 1, or a partial peptide, or an amide or ester thereof, or a salt thereof according to Claim 5.

**13.** A method for quantifying a polypeptide, or an amide or ester thereof, or a salt thereof according to Claim 1, or a partial peptide, or an amide or ester thereof, or a salt thereof according to Claim 5, **characterized by** the use of the antibody according to Claim 12.

**14.** A diagnostic agent comprising the DNA according to Claim 6 or 8, or the antibody according to Claim 12.

**15.** An antisense DNA which comprises a base sequence or a portion thereof, complementary or substantially complementary to a base sequence of the DNA according to Claim 6 or 8, and which has activity capable of inhibiting the expression of said DNA.

**16.** An agent comprising a polypeptide, or an amide or ester thereof, or a salt thereof according to Claim 1, or a partial peptide, or an amide or ester thereof, or a salt thereof according to Claim 5.

**17.** A medicinal drug comprising a polypeptide, or an amide or ester thereof, or a salt thereof according to Claim 1, or a partial peptide, or an amide or ester thereof, or a salt thereof according to Claim 5.

**18.** A method for screening a compound or a salt thereof which promotes or inhibits the activity of a polypeptide, or an amide or ester thereof, or a salt thereof according to Claim 1, or a partial peptide, or an amide or ester thereof, or a salt thereof according to Claim 5, **characterized by** the use of a polypeptide, or an amide or ester thereof, or a salt thereof according to Claim 1, or a partial peptide, or an amide or ester thereof, or a salt thereof according to Claim 5.

**19.** A screening kit for a compound or a salt thereof which promotes or inhibits the activity of a polypeptide, or an amide or ester thereof, or a salt thereof according to Claim 1, or a partial peptide, or an amide or ester thereof, or a salt thereof according to Claim 5, comprising a polypeptide, or an amide or ester thereof, or a salt thereof according to Claim 1, or a partial peptide, or an amide or ester thereof, or a salt thereof according to Claim 5.

**20.** A compound or a salt thereof which promotes or inhibits the activity of a polypeptide, or an amide or ester thereof, or a salt thereof according to Claim 1, or a partial peptide, or an amide or ester thereof, or a salt thereof according to Claim 5, and which is obtained using the screening method according to Claim 18 or the screening kit according to Claim 19.

**21.** A medicinal drug comprising a compound or salt thereof which promotes or inhibits the activity of a polypeptide, or an amide or ester thereof, or a salt thereof according to Claim 1, or a partial peptide, or an amide or ester thereof, or a salt thereof according to Claim 5, and which is obtained using the screening method according to Claim 18 or the screening kit according to Claim 19.

Figure 1

```
ATGAAGCTGG CATTCCTCTT CCTTGGCCCC ATGGCCCTCC TCCTTCTGGC TGGCTATGGC    60
TGTGTCCTCG GTGCCTCCAG TGGGAACCTG CGCACCTTTG TGGGCTGTGC CGTGAGGGAG   120
TTTACTTTCC TGGCCAAGAA GCCAGGCTGC AGGGGCCTTC GGATCACCAC GGATGCCTGC   180
TGGGGTCGCT GTGAGACCTG GGAGAAACCC ATTCTGGAAC CCCCCTATAT TGAAGCCCAT   240
CATCGAGTCT GTACCTACAA CGAGACCAAA CAGGTGACTG TCAAGCTGCC CAACTGTGCC   300
CCGGGAGTCG ACCCCTTCTA CACCTATCCC GTGGCCATCC GCTGTGACTG CGGAGCCTGC   360
TCCACTGCCA CCACGGAGTG TGAGACCATC TGA                                393
```

# Figure 2

```
Met Lys Leu Ala Phe Leu Phe Leu Gly Pro Met Ala Leu Leu Leu Leu
                5                   10                  15
Ala Gly Tyr Gly Cys Val Leu Gly Ala Ser Ser Gly Asn Leu Arg Thr
            20                  25                  30
Phe Val Gly Cys Ala Val Arg Glu Phe Thr Phe Leu Ala Lys Lys Pro
        35                  40                  45
Gly Cys Arg Gly Leu Arg Ile Thr Thr Asp Ala Cys Trp Gly Arg Cys
    50                  55                  60
Glu Thr Trp Glu Lys Pro Ile Leu Glu Pro Pro Tyr Ile Glu Ala His
65                  70                  75                  80
His Arg Val Cys Thr Tyr Asn Glu Thr Lys Gln Val Thr Val Lys Leu
                85                  90                  95
Pro Asn Cys Ala Pro Gly Val Asp Pro Phe Tyr Thr Tyr Pro Val Ala
            100                 105                 110
Ile Arg Cys Asp Cys Gly Ala Cys Ser Thr Ala Thr Thr Glu Cys Glu
        115                 120                 125
Thr Ile End
    130
```

# Figure 3

```
1   M K - L A F L F L G P M A L L L L A G Y G C V L G A S S G N L R T F V G C A V R   VH098489.pro
1   M K T L Q F F F L - - - - - F - - - - - - C C W - - - K A I C C N S - - C E L T   Fsh.pro
1   M E M L Q G L L L - - - - - L L L L S M G G A W - A S R E P L R P W - - C H P I   Lh.pro
1   M T A L - - F L M - - - - - S M L F G L A C G - - - - - - Q A M S F - - C I P T   Tsh.pro

40  E F T F L A K K P G C R G - L R I T T D A C W G R C E T W E - - - K P I L E P P   VH098489.pro
25  N I T I A I E K E E C R F C I S I N T T W C A G Y C Y T R D L V Y K D - - - - P   Fsh.pro
33  N A I L A V E K E G C P V C I T V N T T I C A G Y C P T M M R V L Q A V L - - P   Lh.pro
26  E Y T M H I E R R E C A Y C L T I N T T I C A G Y C M T R D I N G K L F L - - P   Tsh.pro

76  Y I E A H H R V C T Y N E T K Q V T V K L P N C A P G V D P F Y T Y P V A I R C   VH098489.pro
61  A R P K I Q K T C T F K E L V Y E T V R V P G C A H H A D S L Y T Y P V A T Q C   Fsh.pro
71  - - P L P Q V V C T Y R D V R F E S I R L P G C P R G V D P V V S F P V A L S C   Lh.pro
64  K Y A L S Q D V C T Y R D F I Y R T V E I P G C P L H V A P Y F S Y P V A L S C   Tsh.pro

116 D C G A C S T A T T E C E - - - - - - - - - - - - - - - - - - - - T I             VH098489.pro
101 H C G K C D S D S T D C T V R G L G P S Y C S F G E M K E                         Fsh.pro
109 R C G P C R R S T S D C G G P K D H P L T C D H P Q L S G L L - F - L             Lh.pro
104 K C G K C N T D Y S D C I H E A I K T N Y C T K P Q K S Y L V G F S V             Tsh.pro
```

# Figure 4

```
  1 ATGAAGCTGGTATACCTTGTCCTTGGTACTGCGGCCCTCCTTCTGGGTGGCTCTGACTCT    60
  1 MetLysLeuValTyrLeuValLeuGlyThrAlaAlaLeuLeuLeuGlyGlySerAspSer    20

 61 GTCCTCAGCAGCTCCAGCGGGAACCTACACACTTTTGTGGGATGTGCTGTGAGGGAATTC   120
 21 ValLeuSerSerSerSerGlyAsnLeuHisThrPheValGlyCysAlaValArgGluPhe    40

121 ACTTTTGTGGCCAAGAAGCCAGGCTGCAGGGGACTTCGGATCACCACAGATGCCTGCTGG   180
 41 ThrPheValAlaLysLysProGlyCysArgGlyLeuArgIleThrThrAspAlaCysTrp    60

181 GGTCGCTGTGAGACCTGGGAGAAACCCATTCTGGAGCCTCCCTACATAGAAGCCTATCAT   240
 61 GlyArgCysGluThrTrpGluLysProIleLeuGluProProTyrIleGluAlaTyrHis    80

241 CGAGTGTGTACCTACAATGAGACCAGACGGGTGACGGTGAAGCTGCCTAACTGTGCCCCT   300
 81 ArgValCysThrTyrAsnGluThrArgArgValThrValLysLeuProAsnCysAlaPro   100

301 GGAGTCGACCCCTTCTACACCTACCCTATGGCTGTCCGATGTGACTGCGGGGCATGTTCC   360
101 GlyValAspProPheTyrThrTyrProMetAlaValArgCysAspCysGlyAlaCysSer   120

361 ACTGCCACCACTGAGTGTGAGACCATCTGA                                 390
121 ThrAlaThrThrGluCysGluThrIle***                                 130
```

Figure 5

```
1   M K L A F L F L G P M A L L L L A G Y G C V L G A S S G N L R T F V G C A V R E F T F L A K K P G C  Human
1   M K L V Y L V L G T A A L L L - G G S D S V L S S S S G N L H T F V G C A V R E F T F V A K K P G C  Rat

51  R G L R I T T D A C W G R C E T W E K P I L E P P Y I E A H H R V C T Y N E T K Q V T V K L P N C A  Human
50  R G L R I T T D A C W G R C E T W E K P I L E P P Y I E A Y H R V C T Y N E T R R V T V K L P N C A  Rat

101 P G V D P F Y T Y P V A I R C D C G A C S T A T T E C E T I  Human
100 P G V D P F Y T Y P M A V R C D C G A C S T A T T E C E T I  Rat
```

Figure 6

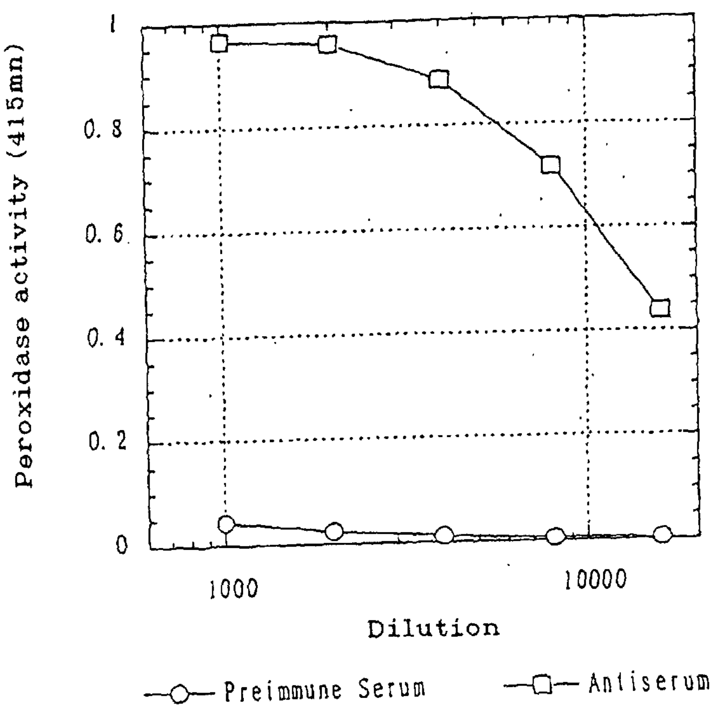
Peroxidase activity (415mn)
1
0.8
0.6
0.4
0.2
0
1000
10000
Dilution
Preimmune Serum
Antiserum

Figure 7

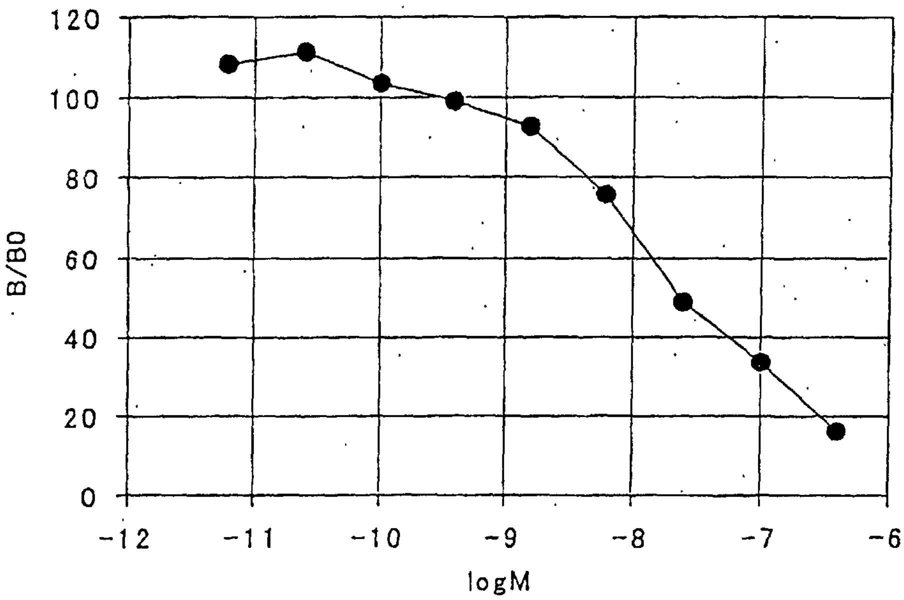

Figure 8

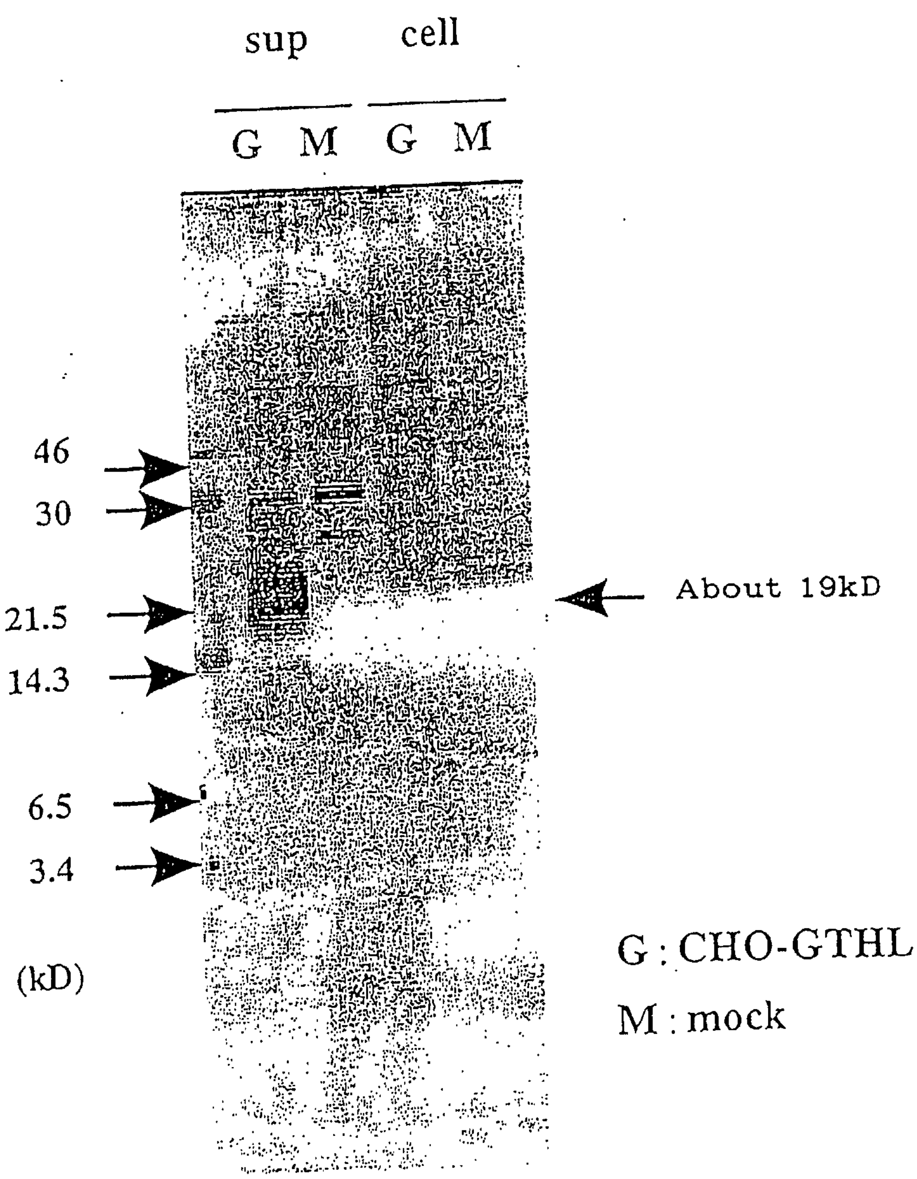

# INTERNATIONAL SEARCH REPORT

International application No.
PCT/JP00/08896

A. CLASSIFICATION OF SUBJECT MATTER
Int.Cl[7] C12N15/16, C07K14/59, C12P21/02, C12Q1/68, C12N1/15, C12N1/19, C12N1/21, C12N5/00, A61K45/00, A61P9/12, A61P9/04, A61P37/04, G01N33/15, G01N33/50, G01N33/53
According to International Patent Classification (IPC) or to both national classification and IPC

B. FIELDS SEARCHED
Minimum documentation searched (classification system followed by classification symbols)
Int.Cl[7] C12N15/00-5/16, C07K14/59, C12P21/02

Documentation searched other than minimum documentation to the extent that such documents are included in the fields searched

Electronic data base consulted during the international search (name of data base and, where practicable, search terms used)
GeneBank/EMBL/DDBJ/GeneSeq
SwissProt/PIR/GeneSeq

C. DOCUMENTS CONSIDERED TO BE RELEVANT

| Category* | Citation of document, with indication, where appropriate, of the relevant passages | Relevant to claim No. |
|---|---|---|
| A | EP, 155655, A1 (FUJISAWA PHARM CO LTD), 25 September, 1985 (25.09.85) & DE, 3584179, G & DK, 850186, A & US, 5019500, A & JP, 61-1396, A | 1-21 |
| A | JP, 10-36285, A (Denki Kagaku Kogyo K. K.), 10 February, 1998 (10.02.98) (Family: none) | 1-21 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 30 January, 2001 (30.01.01) | 06 February, 2001 (06.02.01) |
| Name and mailing address of the ISA/ Japanese Patent Office | Authorized officer |
| Facsimile No. | Telephone No. |

Form PCT/ISA/210 (second sheet) (July 1992)

SEQUENCE LISTING

<110> Takeda Chemical Industries, Ltd.

<120> Novel Polypeptide and its DNA

<130> 2678WOOP

<150> JP 11-358707

<151> 1999-12-17

<150> JP 2000-46825

<151> 2000-02-18

<160> 16

<210> 1

<211> 130

<212> PRT

<213> Human

<400> 1

Met Lys Leu Ala Phe Leu Phe Leu Gly Pro Met Ala Leu Leu Leu Leu
5 10 15

Ala Gly Tyr Gly Cys Val Leu Gly Ala Ser Ser Gly Asn Leu Arg Thr
20 25 30

Phe Val Gly Cys Ala Val Arg Glu Phe Thr Phe Leu Ala Lys Lys Pro
35 40 45

Gly Cys Arg Gly Leu Arg Ile Thr Thr Asp Ala Cys Trp Gly Arg Cys
50 55 60

Glu Thr Trp Glu Lys Pro Ile Leu Glu Pro Pro Tyr Ile Glu Ala His
65 70 75 80

His Arg Val Cys Thr Tyr Asn Glu Thr Lys Gln Val Thr Val Lys Leu
85 90 95

Pro Asn Cys Ala Pro Gly Val Asp Pro Phe Tyr Thr Tyr Pro Val Ala
100 105 110

Ile Arg Cys Asp Cys Gly Ala Cys Ser Thr Ala Thr Thr Glu Cys Glu
115 120 125

Thr Ile
130

<210> 2

<211> 390

<212> DNA

<213> Human

<400> 2

ATGAAGCTGG CATTCCTCTT CCTTGGCCCC ATGGCCCTCC TCCTTCTGGC TGGCTATGGC 60
TGTGTCCTCG GTGCCTCCAG TGGGAACCTG CGCACCTTTG TGGGCTGTGC CGTGAGGGAG 120
TTTACTTTCC TGGCCAAGAA GCCAGGCTGC AGGGGCCTTC GGATCACCAC GGATGCCTGC 180
TGGGGTCGCT GTGAGACCTG GGAGAAACCC ATTCTGGAAC CCCCCTATAT TGAAGCCCAT 240
CATCGAGTCT GTACCTACAA CGAGACCAAA CAGGTGACTG TCAAGCTGCC CAACTGTGCC 300
CCGGGAGTCG ACCCCTTCTA CACCTATCCC GTGGCCATCC GCTGTGACTG CGGAGCCTGC 360
TCCACTGCCA CCACGGAGTG TGAGACCATC 390

<210> 3

<211> 27

<212> DNA

<213> Artificial Sequence

<220>

<223> Primer

<400> 3

CGGAAGAGCA GCATGAAGCT GGCATTC 27

<210> 4

<211> 27

<212> DNA

<213> Artificial Sequence

<220>

<223>

<400> 4

CATGTGCTGC TCACACAGGT GGGTCTG 27

<210> 5

<211> 106

<212> PRT

<213> Human

<400> 5

Ala Ser Ser Gly Asn Leu Arg Thr Phe Val Gly Cys Ala Val Arg Glu
1 5 10 15

Phe Thr Phe Leu Ala Lys Lys Pro Gly Cys Arg Gly Leu Arg Ile Thr
20 25 30

Thr Asp Ala Cys Trp Gly Arg Cys Glu Thr Trp Glu Lys Pro Ile Leu
35 40 45

Glu Pro Pro Tyr Ile Glu Ala His His Arg Val Cys Thr Tyr Asn Glu
50 55 60

Thr Lys Gln Val Thr Val Lys Leu Pro Asn Cys Ala Pro Gly Val Asp
65 70 75 80

Pro Phe Tyr Thr Tyr Pro Val Ala Ile Arg Cys Asp Cys Gly Ala Cys
85 90 95

Ser Thr Ala Thr Thr Glu Cys Glu Thr Ile
100 105

<210> 6

<211> 378

<212> DNA

<213> Human

<400> 6

GCCTCCAGTG GGAACCTGCG CACCTTTGTG GGCTGTGCCG TGAGGGAGTT TACTTTCCTG 60

GCCAAGAAGC CAGGCTGCAG GGGCCTTCGG ATCACCACGG ATGCCTGCTG GGGTCGCTGT 120

GAGACCTGGG AGAAACCCAT TCTGGAACCC CCCTATATTG AAGCCCATCA TCGAGTCTGT 240
ACCTACAACG AGACCAAACA GGTGACTGTC AAGCTGCCCA ACTGTGCCCC GGGAGTCGAC 300
CCCTTCTACA CCTATCCCGT GGCCATCCGC TGTGACTGCG GAGCCTGCTC CACTGCCACC 360
ACGGAGTGTG AGACCATC 378

<210> 7
<211> 116
<212> PRT
<213> Human
<400> 7

Met Asp Tyr Tyr Arg Lys Tyr Ala Ala Ile Phe Leu Val Thr Leu Ser
5 10 15
Val Phe Leu His Val Leu His Ser Ala Pro Asp Val Gln Asp Cys Pro
20 25 30
Glu Cys Thr Leu Gln Glu Asn Pro Phe Phe Ser Gln Pro Gly Ala Pro
35 40 45
Ile Leu Gln Cys Met Gly Cys Cys Phe Ser Arg Ala Tyr Pro Thr Pro
50 55 60
Leu Arg Ser Lys Lys Thr Met Leu Val Gln Lys Asn Val Thr Ser Glu
65 70 75 80
Ser Thr Cys Cys Val Ala Lys Ser Tyr Asn Arg Val Thr Val Met Gly
85 90 95
Gly Phe Lys Val Glu Asn His Thr Ala Cys His Cys Ser Thr Cys Tyr
100 105 110
Tyr His Lys Ser
115

<210> 8
<211> 348
<212> DNA
<213> Human

<400> 8

ATGGATTACT ACAGAAAATA TGCAGCTATC TTTCTGGTCA CATTGTCGGT GTTTCTGCAT 60

GTTCTCCATT CCGCTCCTGA TGTGCAGGAT TGCCCAGAAT GCACGCTACA GGAAAACCCA 120

TTCTTCTCCC AGCCGGGTGC CCCAATACTT CAGTGCATGG GCTGCTGCTT CTCTAGAGCA 180

TATCCCACTC CACTAAGGTC CAAGAAGACG ATGTTGGTCC AAAAGAACGT CACCTCAGAG 240

TCCACTTGCT GTGTAGCTAA ATCATATAAC AGGGTCACAG TAATGGGGGG TTTCAAAGTG 300

GAGAACCACA CGGCGTGCCA CTGCAGTACT TGTTATTATC ACAAATCT 348

<210> 9

<211> 26

<212> DNA

<213> Artificial Sequence

<220>

<223> Primer

<400> 9

AGGCAGCCTG ATAACAGAAG GGAGAG 26

<210> 10

<211> 26

<212> DNA

<213> Artificial Sequence

<220>

<223> Primer

<400> 10

CTTGGGCCAC CAGCCATGAC TGTGCT 26

<210> 11

<211> 129

<212> PRT

<213> Rat

<400> 11

Met Lys Leu Val Tyr Leu Val Leu Gly Thr Ala Ala Leu Leu Leu Gly

1 5 10 15

Gly Ser Asp Ser Val Leu Ser Ser Ser Ser Gly Asn Leu His Thr Phe

20 25 30

Val Gly Cys Ala Val Arg Glu Phe Thr Phe Val Ala Lys Lys Pro Gly

35 40 45

Cys Arg Gly Leu Arg Ile Thr Thr Asp Ala Cys Trp Gly Arg Cys Glu

50 55 60

Thr Trp Glu Lys Pro Ile Leu Glu Pro Pro Tyr Ile Glu Ala Tyr His

65 70 75 80

Arg Val Cys Thr Tyr Asn Glu Thr Arg Arg Val Thr Val Lys Leu Pro

85 90 95

Asn Cys Ala Pro Gly Val Asp Pro Phe Tyr Thr Tyr Pro Met Ala Val

100 105 110

Arg Cys Asp Cys Gly Ala Cys Ser Thr Ala Thr Thr Glu Cys Glu Thr

115 120 125

Ile

<210> 12

<211> 387

<212> DNA

<213> Rat

<400> 12

ATGAAGCTGG TATACCTTGT CCTTGGTACT GCGGCCCTCC TTCTGGGTGG CTCTGACTCT 60

GTCCTCAGCA GCTCCAGCGG GAACCTACAC ACTTTTGTGG GATGTGCTGT GAGGGAATTC 120

ACTTTTGTGG CCAAGAAGCC AGGCTGCAGG GGACTTCGGA TCACCACAGA TGCCTGCTGG 180

GGTCGCTGTG AGACCTGGGA GAAACCCATT CTGGAGCCTC CCTACATAGA AGCCTATCAT 240

CGAGTGTGTA CCTACAATGA GACCAGACGG GTGACGGTGA AGCTGCCTAA CTGTGCCCCT 300

GGAGTCGACC CCTTCTACAC CTACCCTATG GCTGTCCGAT GTGACTGCGG GGCATGTTCC 360

ACTGCCACCA CTGAGTGTGA GACCATC 387

<210> 13

<211> 106
<212> PRT
<213> Rat
<400> 13

Ser Ser Ser Gly Asn Leu His Thr Phe Val Gly Cys Ala Val Arg Glu
1 5 10 15
Phe Thr Phe Val Ala Lys Lys Pro Gly Cys Arg Gly Leu Arg Ile Thr
20 25 30
Thr Asp Ala Cys Trp Gly Arg Cys Glu Thr Trp Glu Lys Pro Ile Leu
35 40 45
Glu Pro Pro Tyr Ile Glu Ala Tyr His Arg Val Cys Thr Tyr Asn Glu
50 55 60
Thr Arg Arg Val Thr Val Lys Leu Pro Asn Cys Ala Pro Gly Val Asp
65 70 75 80
Pro Phe Tyr Thr Tyr Pro Met Ala Val Arg Cys Asp Cys Gly Ala Cys
85 90 95
Ser Thr Ala Thr Thr Glu Cys Glu Thr Ile
100 105

<210> 14
<211> 318
<212> DNA
<213> Rat
<400> 14

AGCTCCAGCG GGAACCTACA CACTTTTGTG GGATGTGCTG TGAGGGAATT CACTTTTGTG 60
GCCAAGAAGC CAGGCTGCAG GGGACTTCGG ATCACCACAG ATGCCTGCTG GGGTCGCTGT 120
GAGACCTGGG AGAAACCCAT TCTGGAGCCT CCCTACATAG AAGCCTATCA TCGAGTGTGT 180
ACCTACAATG AGACCAGACG GGTGACGGTG AAGCTGCCTA ACTGTGCCCC TGGAGTCGAC 240
CCCTTCTACA CCTACCCTAT GGCTGTCCGA TGTGACTGCG GGCATGTTC CACTGCCACC 300
ACTGAGTGTG AGACCATC 318

<210> 15

<211>

<212> DNA

<213> Artificial Sequence

<220> 31

<223> Primer

<400> 15

CTCGAGAGCA GCATGAAGCT GGCATTCCTC T 31

<210> 16

<211>

<212> DNA

<213> Artificial Sequence

<220> 31

<223> Primer

<400> 16

GCTAGCGGCC TCAGATGGTC TCACACTCCG T 31